# EUROPEAN PATENT SPECIFICATION

(11) **EP 3 494 206 B1**
(45) Date of publication and mention of the grant of the patent: **07.07.2021**
(21) Application number: 17758960.3
(22) Date of filing: 02.08.2017
(51) Int. Cl.: C12N 1/20, C12R 1/225, C12R 1/25, A61K 35/00

(54) **LACTIC BACTERIA AND THE USE THEREOF FOR THE PREVENTIVE, INHIBITORY AND/OR REDUCTIVE TREATMENT OF THE FORMATION OF BACTERIAL BIOFILMS**
MILCHSÄUREBAKTERIEN UND DEREN VERWENDUNG ZUR VORBEUGENDEN, INHIBIERENDEN UND ODER REDUZIERENDEN BEHANDLUNG DER BILDUNG VON BAKTERIELLEN BIOFILMEN.
BACTÉRIES LACTIQUES ET LEUR UTILISATION POUR LE TRAITEMENT PRÉVENTIF, INHIBITOIRE ET/OU RÉDUCTIF DE LA FORMATION DE BIOFILMS BACTÉRIENS

(30) Priority: 03.08.2016 IT 201600081420
(43) Date of publication of application: 12.06.2019
(73) Proprietor: Probiotical S.p.A., 28100 Novara (NO) (IT); CRK S.R.L., 20900 Monza (MB) (IT)
(72) Inventor: MOGNA, Giovanni, 28100 Novara (IT)
(74) Representative: Hoffmann Eitle
(86) International application number: PCT/IB2017/054734
(87) International publication number: WO 2018/025204

(56) References cited:
- EP-A1- 2 455 095
- US-A1- 2014 186 409
- MATSUBARA VICTOR HARUO ET AL: "Probiotic lactobacilli inhibit early stages of Candida albicans biofilm development by reducing their growth, cell adhesion, and filamentation", APPLIED MICROBIOLOGY AND BIOTECHNOLOGY, SPRINGER, DE, vol. 100, no. 14, 18 April 2016 (2016-04-18), pages 6415-6426, XP035986577, ISSN: 0175-7598, DOI: 10.1007/S00253-016-7527-3 [retrieved on 2016-04-18]
- VALDÉZ J C ET AL: "Interference of Lactobacillus plantarum with Pseudomonas aeruginosa in vitro and in infected burns: the potential use of probiotics in wound treatment", CLINICAL MICROBIOLOGY AND INFECTION, WILEY-BLACKWELL PUBLISHING LTD, UNITED KINGDOM, SWITZERLAND, vol. 11, no. 6, 1 June 2005 (2005-06-01), pages 472-479, XP002511012, ISSN: 1198-743X, DOI: 10.1111/J.1469-0691.2005.01142.X
- W. MOHAMMEDSAEED ET AL: "Lactobacillus rhamnosus GG Inhibits the Toxic Effects of Staphylococcus aureus on Epidermal Keratinocytes", APPLIED AND ENVIRONMENTAL MICROBIOLOGY, vol. 80, no. 18, 15 September 2014 (2014-09-15), pages 5773-5781, XP055347831, US ISSN: 0099-2240, DOI: 10.1128/AEM.00861-14
- CLAUDIA VUOTTO ET AL: "Probiotics to counteract biofilm-associated infections: promising and conflicting data", INTERNATIONAL JOURNAL OF ORAL SCIENCE, vol. 6, no. 4, 26 September 2014 (2014-09-26), pages 189-194, XP055284950, CN ISSN: 1674-2818, DOI: 10.1038/ijos.2014.52

## Description

The present disclosure relates to selected bacterial strains belonging to the genus *Lactobacillus* and the use thereof for the treatment of bacterial biofilms and, specifically, for preventive treatment aimed at inhibiting and/or reducing the formation of bacterial biofilms. In particular, the present disclosure relates to the following bacterial strains belonging to the genus *Lactobacillus,* selected from the group comprising or, alternatively, consisting of *Lactobacillus plantarum* LMC1 (DSM 32252), *Lactobacillus reuteri* LMC3 (DSM 32253), *Lactobacillus paracasei* LMC4 (DSM 32254), *Lactobacillus reuteri* LMC5 (DSM 32255), *Lactobacillus rhamnosus* LMC6 (DSM 32256), *Lactobacillus rhamnosus* LMC7 (DSM 32257), *Lactobacillus paracasei* LMC8 (DSM 32258), *Lactobacillus reuteri* LMC9 (DSM 32259) and *Lactobacillus rhamnosus* LMC10 (DSM 32260), and the use thereof for the treatment of bacterial biofilms and, specifically, for preventive treatment aimed at inhibiting and/or reducing the formation of bacterial biofilms. Furthermore, the present disclosure relates to a pharmaceutical composition, a composition for a medical device or a composition for dietary supplements comprising a mixture which comprises or, alternatively, consists of one or more of the bacterial strains specified above and, optionally, pharmaceutical or food grade technological additives and/or excipients, for the treatment of bacterial biofilms and, specifically, for preventive treatment aimed at inhibiting and/or reducing the formation of bacterial biofilms.

In the biomedical context, a so-called biofilm is made up of a community of microorganisms anchored to a biological or non-biological surface and incorporated/enclosed in an extracellular exopolysaccharide matrix (1). Biofilm formation (which takes place under the control of specific bacterial genes responsible for its production) is a process that has been compared to cellular differentiation in multicellular organisms. A typical example of a biofilm is represented by the one that forms when bacteria adhere to a biological or non-biological surface and anchor themselves to it, growing and dividing so as to form two coating layers made up of an extracellular matrix of polymeric substances, polysaccharides in particular. Said extracellular matrix on the surface of the bacteria incorporated within the biofilm effectively protects them against the action of external agents such as, for example, antibiotics (2). Many studies have in fact demonstrated that a biofilm can increase the resistance of microorganisms to antimicrobial agents by over one hundred times compared to that of the same bacteria in planktonic form (3, 4); consequently, it is very difficult to eliminate bacteria within a biofilm, because there are no existing molecules capable of effectively penetrating this biological structure. It is estimated that around 80% of the world microbial mass is present in a biofilm state and that the aforesaid microbial biofilms are the cause of more than 75% of the total microbial infections that may be found in human beings (5). A bacterial biofilm can form on the outer surface of or inside a living organism (for example, human or animal) in or in proximity to or because of lesions of various types associated with an environment that is not perfectly sterile, as in the case, for example, of prostheses. Furthermore, bacterial biofilms can also form on abiotic surfaces that are not perfectly sterilised, for example, intravascular catheters and prosthetic implants (6) or in the case for example of bones, bone tissue and insertions of prostheses in bone tissue. The microorganisms within the biofilm on such artificial surfaces often derive from the skin flora of the patients themselves or of medical personnel during the insertion or implantation of the device. Solely by way of non-limiting example, the predominant microorganisms include, in particular, *Coagulase-Negative Staphylococci, Staphylococcus aureus,* various species of *Pseudomonas, Enterococcus, Stenotrophomonas,* and *Candida* on intravascular catheters; *Escherichia coli, Enterococcus, Pseudomonas, Klebsiella, Enterobacter, Proteus mirabilis* and *Candida* on urinary catheters; and *Staphylococcus aureus, Staphylococcus emidermitis and Propionibacterium acnes* on hip replacement implants (6).
Since such types of implants often remain in the body of patients for a long period of time, the formation of biofilms by these harmful microorganisms may give rise to serious localised and/or systemic infections that are difficult to eradicate, such as, for example, osteomyelitis, which is an infectious process that simultaneously affects the bones and bone marrow (6). Oral biofilms also constitute a breeding ground for microorganisms which can spread through transient bacteraemia, as demonstrated by the different types of biofilms isolated from oral cavity infections (7). The detachment and consequent haematogenous dissemination of the bacteria present in the biofilm has been associated with some forms of infectious endocarditis, acute bacterial myocarditis, brain abscesses, liver abscesses, lung abscesses and thrombosis of the cavernous sinus (8-13). The hypothesis that oral biofilms may have consequences on systemic health is supported by cross-sectional studies which report elevated levels of markers of systemic inflammations in patients with periodontitis (14, 15). Convincing evidence supports the systemic-oral link between periodontitis and cardiovascular and cerebrovascular diseases and diabetes mellitus, all having an inflammatory etiology. Furthermore, associations between periodontitis and cardiovascular diseases have been demonstrated, irrespective of common risk factors as factors such as smoking, age, education, body mass index and lifestyle (16).
However, it is worth noting that, contrary to what has been described above, there also exist biofilms, naturally present in the gastrointestinal and/or female urogenital tract, that contain beneficial microorganisms, such as, for example, lactobacilli and *Streptococcus thermophilus,* which are capable of performing a protective role in an organism, because such a structure (biofilm) facilitates the long-term colonisation of regions and the persistence of these bacteria in the organism (17).
The negative impact on human health of bacterial microbial biofilms produced by pathogenic bacteria, associated, among other things, with the reduction in the effectiveness of antibiotics, has thus stimulated a quest for new biologically active products that are able to act effectively as anti-biofilm agents capable of inhibiting their formation (preventive action) and/or blocking and/or reducing their growth/development (therapeutic action), thereby enabling an optimal application of the necessary/desired antimicrobial/antibacterial treatment (for example, antibiotics).

Matsubara et al, Appl Microbiol Biotechnol (2016) discloses that Lactobacillus species (rhamnosus, casei, acidophilus) antagonize Candida albicans biofilm formation through cell-cell interaction and secretion of exometabolites that inhibit their filamentation.

US2014186409 pertains to lactic acid bacteria (Lactobacillus species) which aggregate with infectious bacterial strains (Staphylococcus aureus or Pseudomonas aeruginosa), in particular by coaggregating with them and thus reducing the local concentration of the microorganisms, inhibiting their growth or even killing them or preventing the formation of a biofilm.

However, to the Applicant's knowledge, no products have yet been identified/produced to date which are capable of achieving the above in a completely satisfactory manner.

Thus, there continues to be a strong demand on the part of practitioners in this field to have at their disposal products, compositions and formulations capable of acting effectively as anti-bacterial biofilm agents so as to inhibit biofilm formation and/or block and/or reduce the growth/development thereof, thereby enabling an optimal application of the necessary/desired antibacterial treatment (for example, antibiotics).

The aim of the present invention is to provide an adequate response to the above-described technical problem.

The Applicant has now unexpectedly found that a suitable group of specific selected lactobacilli of human origin is capable of providing the desired response to the above-described technical problem.

An object of the present invention is one or more bacterial strains of human origin belonging to the genus Lactobacillus, as set forth in the appended independent claim.

A further object of the present invention is one or more bacterial strains as defined in the claims for use as a medicament.

A further object of the present invention is one or more bacterial strains of human origin belonging to the genus Lactobacillus, as defined in the claims,for use in the treatment of bacterial biofilms and, specifically, for preventive treatment aimed at inhibiting and/or reducing the formation of bacterial biofilms, as set forth in the appended claims.

A further object of the present invention is a pharmaceutical composition or a composition for a medical device or a composition for dietary supplements comprising a mixture which comprises or, alternatively, consists of one or more of the bacterial strains specified above and, optionally, pharmaceutical or food grade technological additives and/or excipients, as set forth in the appended independent claim.

A further object of the present invention is a pharmaceutical composition or a composition for a medical device or a composition for dietary supplements comprising a mixture which comprises or, alternatively, consists of one or more of the bacterial strains specified above and, optionally, pharmaceutical or food grade technological additives and/or excipients, for use in the treatment of bacterial biofilms and, specifically, for preventive treatment aimed at inhibiting and/or reducing the formation of bacterial biofilms, as set forth in the appended independent claim.

Further objects of the present invention are the uses of the aforesaid lactobacilli, as set forth in the appended claims. In particular, object of the present invention is a composition comprising microorganisms belonging to one or more of the following strains:
*Lactobacillus plantarum,* with deposit number DSM 32252 [otherwise indicated as LMC1];
*Lactobacillus paracasei,* with deposit number DSM 32254 [otherwise indicated as LMC4];
*Lactobacillus rhamnosus,* with deposit number DSM 32256 [otherwise indicated as LMC6];
*Lactobacillus rhamnosus,* with deposit number DSM 32257 [otherwise indicated as LMC7];
*Lactobacillus paracasei,* deposited as DSM 32258 [otherwise indicated as LMC8]
for use in the treatment or prevention of biofilm formation by harmful bacterial strains such as, preferably, those selected from among *Candida, Escherichia coli, Klebsiella, Proteus mirabilis* and *Propionibacterium acnes, S.aureus,* and of disorders or pathologies correlated to said biofilm formation.

Preferred embodiments of the present invention are set forth in the appended dependent claims.

The preferred embodiments of the present invention disclosed in the following description are here illustrated solely by way of example and in no way limit the broad scope of application of the present invention, which will appear immediately clear to the person skilled in the art.

Table 1 illustrates the percentage of inhibition of biofilm production by *S. aureus* obtained using the lactobacilli of the present invention.

**Table 1**

| Supernatant | % inhibition |
|---|---|
| LMC1 | 84 |
| LMC4 | 82 |
| LMC6 | 54 |
| LMC7 | 62 |
| LMC8 | 87 |

Figure 1 shows a 3D image of the biofilm in the supernatant of *S. aureus* (control). The biofilm was stained with the FilmTracerTM LIVE/DEAD® Biofilm Viability Kit; the green stain (SYTO9) represents live cells; the red stain (Propidium Iodide) represents dead cells.

Figure 2 shows a cross-sectional image of the biofilm in the supernatant of *S. aureus* (control).

Figure 3 shows a sectional image of the biofilm produced by *S. aureus* after incubation in the supernatant of LMC8; substantially, very little/no biofilm production.

### Characterisation of the strain Lactobacillus plantarum (DSM 32252)

Strain: *Lactobacillus plantarum*
Internal identification number - ID: ID 1952
Probiotical commercial code: LMC-1
Deposit number in DSMZ international collection: DSM 32252

### Strain Characterisation

### Origin

The strain *Lactobacillus plantarum* was isolated and analysed in the laboratories of Biolab Research srl, a subsidiary of the Mofin Alce Group, an affiliate of Probiotical S.p.A.

### Biochemical characterisation

### 1) Table 2 shows the sugar fermentation profile (API 50 CHL, bioMérieux)

**Table 2**

| **N.** | Sugars | Results | **N.** | Sugars | Results |
|---|---|---|---|---|---|
| **0** | Control | - | **25** | Esculin ferric citrate | + |
| **1** | Glycerol | - | **26** | Salicin | + |
| **2** | Erythritol | - | **27** | D-Cellobiose | + |
| **3** | D-Arabinose | - | **28** | D-Maltose | + |
| **4** | L-Arabinose | + | **29** | D-Lactose | + |
| **5** | D-Ribose | + | **30** | D-Melibiose | + |
| **6** | D-Xylose | - | **31** | D-Sucrose | + |
| **7** | L-Xylose | - | **32** | D-Trehalose | + |
| **8** | Adonitol | - | **33** | Inulin | - |
| **9** | Methyl-βD-Xylopyranoside | - | **34** | D-Melezitose | + |
| **10** | D-Galactose | + | **35** | D-Raffinose | + |
| **11** | D-Glucose | + | **36** | Starch | - |
| **12** | D-Fructose | + | **37** | Glycogen | - |
| **13** | D-Mannose | + | **38** | Xylitol | - |
| **14** | L-Sorbose | - | **39** | Gentiobiose | + |
| **15** | L-Rhamnose | + | **40** | D-Turanose | + |
| **16** | Dulcitol | - | **41** | D-Lyxose | - |
| **17** | Inositol | - | **42** | D-Tagatose | - |
| **18** | D-Mannitol | + | **43** | D-Fucose | - |
| **19** | D-Sorbitol | + | **44** | L-Fucose | - |
| **20** | Methyl-aD-Mannopyranoside | - | **45** | D-Arabitol | - |
| **21** | Methyl-aD-Glucopyranoside | - | **46** | L-Arabitol | - |
| **22** | N-Acetyl-Glucosamine | + | **47** | Potassium gluconate | + |
| **23** | Amygdalin | + | **48** | Potassium 2-Ketogluconate | - |
| **24** | Arbutin | + | **49** | Potassium 5-Ketogluconate | - |

### 2) Table 3 shows the enzymatic profile (API Zym, bioMérieux)

**Table 3**

| N. | Substrate | Enzymes analysed | Type of enzyme | Activity (nanomoles) |
|---|---|---|---|---|
| **1** | Negative control | - | - | - |
| **2** | 2-naphthyl-phosphate | Alkaline phosphatase | PHOSPHATASE | 5 |
| **3** | 2-naphthyl butyrate | Esterase (C 4) | | 5 |
| **4** | 2-naphthyl caprylate | Esterase lipase (C 8) | LIPASE | 5 |
| **5** | 2-naphthyl myristate | Lipase (C 14) | | - |
| **6** | L-leucyl-2-naphthylamide | Leucine arylamidase | PROTEASE | ≥40 |
| **7** | L-valyl-2-naphthylamide | Valine arylamidase | | 30 |
| **8** | L-cystyl-2-naphthylamide | Cystine arylamidase | | ≥40 |
| **9** | N-benzoyl-DL -arginine-2-naphthyl amide | Trypsin | | - |
| **10** | N-glutaryl-phenylalanine-2-naphthyl amide | α-chymotrypsin | | - |
| **11** | 2-naphthyl-phosphate | Acid phosphatase | PHOSPHATASE | 30 |
| **12** | Naphthol-AS-BI-phosphate | Naphthol-AS-BI-phosphohydrolase | | 10 |
| **13** | 6-Br-2-naphthyl-αD-galactopyranoside | α-galactosidase | OXIDASE | 10 |
| **14** | 2-naphthyl-βD-galactopyranoside | β-galactosidase | | ≥40 |
| **15** | Naphthol-AS-BI-βD-glucoronide | β-glucuronidase | | - |
| **16** | 2-naphthyl-αD-glucopyranoside | α-glucosidase | | 20 |
| **17** | 6-Br-2-naphthyl-βD-glucopyranoside | β-glucosidase | | 20 |
| **18** | 1-naphthyl-N-acetyl-βD-*glucosaminide* | N-acetyl-β-glucosaminidase | | ≥40 |
| **19** | 6-Br-2-naphthyl-αD-mannopyranoside | α-mannosidase | | - |
| **20** | 2-naphthyl-αL-fucopyranoside | α-fucosidase | | - |

### 3) Protein profile (Electrophoresis on polyacrylamide gel)

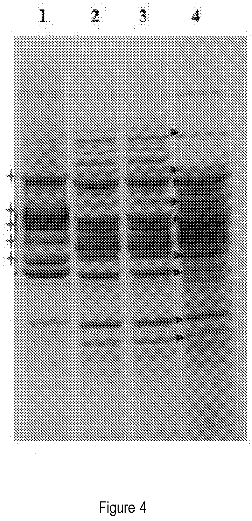

Figure 4 shows the protein profile (electrophoresis on polyacrylamide gel) of the strain concerned. Lines 1-2 show the protein profiles of the Master Cell Bank cultures of the strain; (see lines); as regards lines 1-2 and 3, the profile of the strain is characteristic of the species *Lactobacillus plantarum* (see arrows). In lines 4 and 1-2 the protein profile of the strain is different from the one obtained from a strain belonging to a different species (*Lactobacillus paracasei* DSM 32258; see crosses).

### Molecular characterisation

### Species-specific classification:

### 4) Polymerase chain reaction (PCR) using the primers PLAN-F/ P-REV

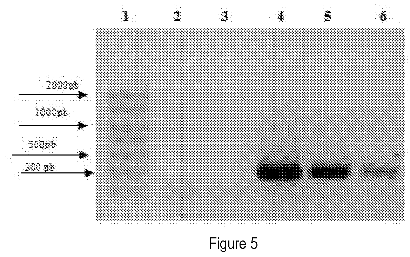

Figure 5 shows the positive reaction for *Lactobacillus plantarum,* where:
1. PCR Marker: Sigma 50-2000 bp
2. Blank: No DNA
3. Negative reference: *L. paracasei* DSM 5622
4. Positive reference: *L. plantarum* DSM 20174
5. Strain: *L. plantarum* LMC-1 ID 1952
6. Strain: *L. plantarum* LMC-1 ID 1952

### 5) 16S rDNAgene sequencing

The results were obtained with the Blast program (http://blast.ncbi.nlm.nih.gov/Blast.cgi).

| Length=1542 | | |
|---|---|---|
| Sequences producing significant alignments: | Score (Bits) | E Value |
| gb\|CP012343.1\| Lactobacillus plantarum strain ZS2058, complet... | 2769 | 0.0 |
| gb\|KT2156161.\| Lactobacillus pentosus strain FL0421 16S ribos... | 2769 | 0.0 |
| gb\|CP010528.1\| Lactobacillus plantarum strain B21, complete g... | 2769 | 0.0 |
| gb\|CP005942.2\| Lactobacillus plantarum subsp. plantarum P-8, ... | 2769 | 0.0 |
| gb\|CP004406.1\| Lactobacillus plantarum DOMLa, complete genome | 2769 | 0.0 |
| ref\|NR_075041.1\| Lactobacillus plantarum WCFS1 strain WCFS1 1... | 2769 | 0.0 |
| gb\|JX025073.1\| Lactobacillus plantarum strain IR BL0076 16S r... | 2769 | 0.0 |
| emb\|AL935263.2\| Lactobacillus plantarum WCFS1 complete genome | 2769 | 0.0 |
| emb\|FR871789.1\| Lactobacillus pentosus MP-10 draft genome, an... | 2769 | 0.0 |
| gb\|GU552552.1\| Lactobacillus plantarum strain KW30 16S riboso... | 2769 | 0.0 |

Score: Number used to evaluate the biological relevance of an identification. In sequence alignments, the score is a numerical value describing the overall quality of an alignment. Higher numbers correspond to greater similarity.
E-value (expected value): a value which, if correctly interpreted by a researcher, will indicate the likelihood of a score indicating a correlation between the two biological sequences. The lower the E-value, the more significant the score.

### Fingerprinting Profile

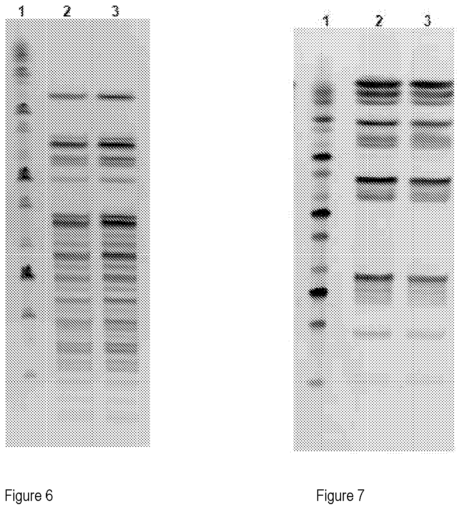

6) Figures 6 and 7 show the pulsed-field electrophoresis (PFGE) with the Notl enzyme (Figure 6) and Sfil enzyme (Figure 7), where:
1. Marker: Sigma 50-1,000 kb
2. *L. plantarum* LMC-1 ID 1952 - origin - Master Cell Bank
3. *L. plantarum* LMC-1 ID 1952 - 6th sub-culture - Master Cell Bank

### Biological characterisation

### 7) Table 4 shows the antibiotic resistance profile (E-test, ABBiodisk)

**Table 4**

| Antibiotic class | MIC* µg/ml | Bacterial strains | | | |
|---|---|---|---|---|---|
| | | *Lactobacillus plantarum* LMC-1 ID 1952 DSM 32252 | *Commercial Lactobacillus*^ | *Commercial Bifidobacterium*^ | EFSA Limit 2012 |
| aminoglycosides | Gentamicin | 4 | 6 | 48 | 16 |
| | Streptomycin | 16 | 12 | 64 | n.r |
| | Kanamycin | 64 | 128 | n.d. | 64 |
| quinolones | Ciprofloxacin | >32 | 1 | 4 | - |
| glycopeptides | Vancomycin | >256 | >256 | 0.38 | n.r |
| lincosamides | Clindamycin | 2 | 0.50 | 0.047 | 2 |
| macrolides | Azithromycin | 2 | 0.38 | 0.75 | - |
| | Clarithromycin | 0.25 | 0.047 | 0.032 | - |
| | Erythromycin | 0.75 | 0.032 | 0.094 | 1 |
| oxazolidinone** | Linezolid | 1.5 | 2 | 0.38 | 4 |
| rifamicin | Rifampicin | 0.25 | 0.094 | 0.094 | - |
| strepogramin*** | Quinupristin/Dalfopristin | 0.38 | 1 | 0.25 | 4 |
| tetracyclines | Cloramphenicol | 2 | 3 | 0.75 | 8 |
| | Tetracycline | 12 | 0.50 | 8 | 32 |
| β-lactam | Amoxicillin | 0.19 | 0.50 | 0.064 | - |
| | Ampicillin | 0.032 | 0.50 | 0.016 | 2 |
| | Cefoxitin | 64 | >256 | 1 | - |
| | Cefuroxime | 0.064 | 1.5 | 0.25 | - |
| | Imipenem | 0.047 | 2 | 0.25 | - |

| | | | | | |
|---|---|---|---|---|---|
| ^ The commercial strains were used as a reference. The strains are not identified in this document for ethical reasons. The data are available on request * MIC (Minimum Inhibitory Concentration) assessment of the inhibition loop in agar with Etest strips (ABBiodisk). n.r. not required / n.d. not determined. ** included in EFSA 2005 *** included in EFSA 2008 | | | | | |

### 8) Table 5 shows the resistance to biological fluids (simulated gastric juice, simulated pancreatic juice and bile salts)

**Table 5**

| Strains | Biological fluids | *After different contact times (in. minutes)*^ | | | *In the presence of bile in the medium^^* |
|---|---|---|---|---|---|
| | | 5' | 30' | 60' | |
| *Lactobacillus plantarum* LMC-1 ID 1952 | Simulated gastric juice | 0.2 | 0 | 0 | |
| | Simulated pancreatic | 98 | 93 | 89 | |
| DSM 32252 | secretion | | | | |
| | Bile salts | | | | 100 |
| Commercial Lactobacillus* | Simulated gastric juice | 90 | 30 | 19 | |
| | Simulated pancreatic secretion | 88 | 80 | 73 | |
| | Bile salts | | | | 55 |
| Commercial Bifidobacterium* | Simulated gastric juice | 90 | 65 | 25 | |
| | Simulated pancreatic secretion | 88 | 65 | 40 | |
| | Bile salts | | | | 4 |

| | | | | | |
|---|---|---|---|---|---|
| * The commercial strains were used as a reference. The strains are not identified in this document for ethical reasons. The data are available on request. ^ Table 5 shows the percentage of survival of the probiotic strains in two different types of biological fluids; simulated gastric juice and pancreatic secretion at 37 ° C after different contact times (5, 30 and 60 minutes). ^^ The results of survival in the presence of bile secretion were obtained by comparing the number of colonies growing in the specific medium "with" and "without" the addition of bile salts. | | | | | |

### Characterisation of the strain Lactobacillus paracasei (DSM 32254)

Strain: *Lactobacillus paracasei*
Internal identification number - ID: ID 1953
Probiotical commercial code: LMC-4
Deposit number in DSMZ international collection: DSM 32254

### Strain characterisation

### Origin

The strain *Lactobacillus paracasei* was isolated and analysed in the laboratories of Biolab Research srl, a subsidiary of the Mofin Alce Group, an affiliate of Probiotical S.p.A.

### Biochemical characterisation

### 1) Table 6 shows the sugar fermentation profile (API 50 CHL, bioMérieux)

**Table 6**

| **N.** | Sugars | Results | **N.** | Sugars | Results |
|---|---|---|---|---|---|
| **0** | Control | - | **25** | Esculin ferric citrate | + |
| **1** | Glycerol | - | **26** | Salicin | + |
| **2** | Erythritol | - | **27** | D-Cellobiose | + |
| **3** | D-Arabinose | - | **28** | D-Maltose | + |
| **4** | L-Arabinose | - | **29** | D-Lactose | + |
| **5** | D-Ribose | + | **30** | D-Melibiose | - |
| **6** | D-Xylose | - | **31** | D-Sucrose | + |
| **7** | L-Xylose | - | **32** | D-Trehalose | + |
| **8** | Adonitol | - | **33** | Inulin | - |
| **9** | Methyl-βD-Xylopyranoside | - | **34** | D-Melezitose | + |
| **10** | D-Galactose | + | **35** | D-Raffinose | - |
| **11** | D-Glucose | + | **36** | Starch | - |
| **12** | D-Fructose | + | **37** | Glycogen | - |
| **13** | D-Mannose | + | **38** | Xylitol | - |
| **14** | L-Sorbose | + | **39** | Gentiobiose | + |
| **15** | L-Rhamnose | - | **40** | D-Turanose | + |
| **16** | Dulcitol | - | **41** | D-Lyxose | - |
| **17** | Inositol | - | **42** | D-Tagatose | + |
| **18** | D-Mannitol | + | **43** | D-Fucose | - |
| **19** | D-Sorbitol | + | **44** | L-Fucose | - |
| **20** | Methyl-αD-Mannopyranoside | - | **45** | D-Arabitol | - |
| **21** | Methyl-αD-Glucopyranoside | - | **46** | L-Arabitol | - |
| **22** | N-Acetyl-Glucosamine | + | **47** | Potassium gluconate | + |
| **23** | Amygdalin | + | **48** | Potassium 2-Ketogluconate | - |
| **24** | Arbutin | + | **49** | Potassium 5-Ketogluconate | - |

### 2) Table 7 shows the enzymatic profile (API Zym, bioMérieux)

**Table 7**

| N. | Substrate | Enzymes analysed | Type of enzyme | Activity (nanomoles) |
|---|---|---|---|---|
| **1** | Negative control | - | - | - |
| **2** | 2-naphthyl-phosphate | Alkaline phosphatase | PHOSPHATASE | 5 |
| **3** | 2-naphthyl butyrate | Esterase (C 4) | LIPASE | 5 |
| **4** | 2-naphthyl caprylate | Esterase lipase (C 8) | | 10 |
| **5** | 2-naphthyl myristate | Lipase (C 14) | | - |
| **6** | L-leucyl-2-naphthylamide | Leucine arylamidase | PROTEASE | ≥40 |
| **7** | L-valyl-2-naphthylamide | Valine arylamidase | | ≥40 |
| **8** | L-cystyl-2-naphthylamide | Cystine arylamidase | | 10 |
| **9** | N-benzoyl-DL-arginine-2-naphthyl amide | Trypsin | | - |
| **10** | N-glutaryl-phenylalanine-2-naphthyl amide | α-chymotrypsin | | 10 |
| **11** | 2-naphthyl-phosphate | Acid phosphatase | PHOSPHATASE | ≥40 |
| **12** | Naphthol-AS-BI-phosphate | Naphthol-AS-BI-phosphohydrolase | | ≥40 |
| **13** | 6-Br-2-naphthyl-αD-galactopyranoside | α-galactosidase | OXIDASE | - |
| **14** | 2-naphthyl-βD-galactopyranoside | β-galactosidase | | ≥40 |
| **15** | Naphthol-AS-BI-βD-glucoronide | β-glucuronidase | | - |
| **16** | 2-naphthyl-αD-glucopyranoside | α-glucosidase | | ≥40 |
| **17** | 6-Br-2-naphthyl-βD-glucopyranoside | β-glucosidase | | 20 |
| **18** | 1-naphthyl-N-acetyl-βD-glucosaminide | N-acetyl-β-glucosaminidase | | - |
| **19** | 6-Br-2-naphthyl-αD-mannopyranoside | α-mannosidase | | - |
| **20** | 2-naphthyl-αL-fucopyranoside | α-fucosidase | | 5 |

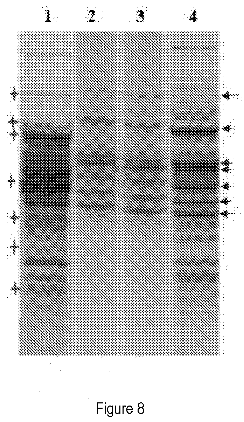

### 3) Figure 8 shows the protein profile (electrophoresis on polyacrylamide gel), where:

1. Negative reference: *L. plantarum* LP01 - LMG P-21021
2. Strain: *L. paracasei* LMC-4 (ID 1953) - Master Cell Bank (origin)
3. Strain: *L. paracasei* LMC-4 (ID 1953) - Master Cell Bank (sub-culture 13#6)
4. Positive reference: *L.paracasei* LPC 01 - CNCM I-1390

Lines 2-3 show the protein profiles of the Master Cell Bank cultures of the strain; (see lines); in lines 1 and 2-3 the profile of the strain is characteristic of the species Lactobacillus paracasei (see arrows). As regards lines 4 and 2-3, the protein profile of the strain is different from the one obtained from a strain belonging to a different species (Lactobacillus plantarum LMG P-21021; see crosses).

### Molecular characterisation

### Species-specific classification:

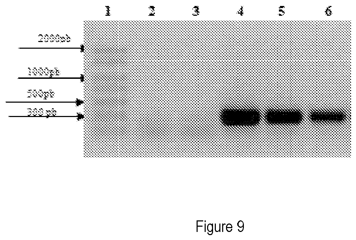

4) Figure 9 shows the Polymerase Chain Reaction (PCR) using the primers W2/Y2. The reaction is positive for *Lactobacillus paracasei,* where:
1. PCR Marker: Sigma 50-2000 bp
2. Blank: No DNA
3. Negative reference: *L. casei* DSM 20011
4. Positive reference: *L. paracasei* DSM 5622
5. Strain: *L.paracasei* LMC-4 ID 1953
6. Strain: *L. paracasei* LMC-4 ID 1953

### 5) 16S rDNA gene sequencing

The results were obtained with the Blast program (http://blast.ncbi.nlm.nih.gov/Blast.cgi).

| Length=1551 | | |
|---|---|---|
| Sequences producing significant alignments: | Score (Bits) | E Value |
| gb\|CP013921.1\| Lactobacillus paracasei strain KL1, complete g... | 2780 | 0.0 |
| gb\|CP012148.1\| Lactobacillus paracasei strain L9, complete ge... | 2780 | 0.0 |
| gb\|CP012197.1\| Lactobacillus paracasei strain CAUH35, complet... | 2780 | 0.0 |
| gb\|CP001084.2\| Lactobacillus casei str. Zhang, complete genome | 2780 | 0.0 |
| gb\|CP006690.1\| Lactobacillus casei 12A, complete genome | 2780 | 0.0 |
| gb\|CP007122.1\| Lactobacillus paracasei N1115, complete genome | 2780 | 0.0 |
| gb\|CP002391.1\| Lactobacillus paracasei subsp. paracasei 8700:... | 2780 | 0.0 |
| dbj\|AP012541.1\| Lactobacillus paracasei subsp. paracasei JCM ... | 2780 | 0.0 |
| gb\|CP005496.1\| Lactobacillus casei LOCK919, complete genome | 2780 | 0.0 |
| ref\|NR_075032.1\| Lactobacillus casei ATCC 334 strain ATCC 334... | 2780 | 0.0 |

Score: Number used to evaluate the biological relevance of an identification. In sequence alignments, the score is a numerical value describing the overall quality of an alignment. Higher numbers correspond to greater similarity.
E-value (expected value): a value which, if correctly interpreted by a researcher, will indicate the likelihood of a score indicating a correlation between the two biological sequences. The lower the E-value, the more significant the score.

### Fingerprinting Profile

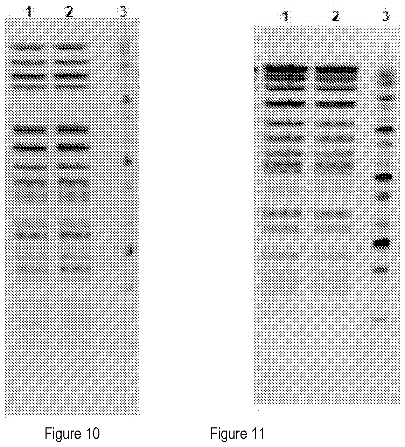

6) Figures 10 and 11 show the pulsed-field electrophoresis (PFGE) with the Notl enzyme(Figure 10) and Sfil enzyme (Figure 11), where:
1. *L. paracasei* LMC-4 (ID 1953) - culture origin - Master Cell Bank
2. *L paracasei* LMC-4 (ID 1953) - 6th sub-culture - Master Cell Bank
3. Electrophoretic Marker: Sigma 50-1,000 kb

### Biological characterisation

### 7) Table 8 shows the antibiotic resistance profile (E-test, ABBiodisk)

**Table 8**

| Antibiotic class | MIC* µg/ml | Bacterial strains | | | |
|---|---|---|---|---|---|
| | | *Lactobacillus paracasei* LMC-4 ID 1953 DSM 32254 | *Commercial Lactobacillus*^ | *Commercial Bifidobacterium* ^ | EFSA Limit 2012 |
| aminoglycosides | Gentamicin | 2 | 6 | 48 | 32 |
| | Streptomycin | 12 | 12 | 64 | 64 |
| | Kanamycin | 24 | 128 | n.d. | 64 |
| quinolones | Ciprofloxacin | 0.75 | 1 | 4 | - |
| glycopeptides | Vancomycin | >256 | >256 | 0.38 | n.r |
| lincosamides | Clindamycin | 0.064 | 0.50 | 0.047 | 1 |
| macrolides | Azithromycin | 0.50 | 0.38 | 0.75 | - |
| | Clarithromycin | 0.047 | 0.047 | 0.032 | - |
| | Erythromycin | 0.125 | 0.032 | 0.094 | 1 |
| oxazolidinone** | Linezolid | 0.38 | 2 | 0.38 | 4 |
| rifamicin | Rifampicin | 0.016 | 0.094 | 0.094 | - |
| strepogramin*** | Quinupristin/Dalfopristin | 0.094 | 1 | 0.25 | 4 |
| tetracyclines | Cloramphenicol | 1.5 | 3 | 0.75 | 4 |
| | Tetracycline | 0.19 | 0.50 | 8 | 4 |
| β-lactam | Amoxicillin | 0.25 | 0.50 | 0.064 | - |
| | Ampicillin | 0.094 | 0.50 | 0.016 | 4 |
| | Cefoxitin | >256 | >256 | 1 | - |
| | Cefuroxime | 0.75 | 1.5 | 0.25 | - |
| | Imipenem | 1.5 | 2 | 0.25 | - |

| | | | | | |
|---|---|---|---|---|---|
| ^ The commercial strains were used as a reference. The strains are not identified in this document for ethical reasons. The data are available on request * MIC (Minimum Inhibitory Concentration) assessment of the inhibition loop in agar with Etest strips (ABBiodisk). n.r. not required / n.d. not determined. ** included in EFSA 2005 *** included in EFSA 2008 | | | | | |

### 8) Table 9 shows the resistance to biological fluids (simulated gastric juice, simulated pancreatic juice and bile salts)

**Table 9**

| Strains | Biological fluids | *After different contact times (in minutes)*^ | | | *In the presence of bile in the medium^^* |
|---|---|---|---|---|---|
| | | 5' | 30' | 60' | |
| *Lactobacillus paracasei* LMC-4 ID 1953 DSM 32254 | Simulated gastric juice | 0 | 0 | 0 | |
| | Simulated pancreatic secretion | 100 | 100 | 100 | |
| | Bile salts | | | | 100 |
| Commercial Lactobacillus* | Simulated gastric juice | 90 | 30 | 19 | |
| | Simulated pancreatic secretion | 88 | 80 | 73 | |
| | Bile salts | | | | 55 |
| Commercial Bifidobacterium* | Simulated gastric juice | 90 | 65 | 25 | |
| | Simulated pancreatic secretion | 88 | 65 | 40 | |
| | Bile salts | | | | 4 |

| | | | | | |
|---|---|---|---|---|---|
| * The commercial strains were used as a reference. The strains are not identified in this document for ethical reasons. The data are available on request. ^ Table 9 shows the percentage of survival of the probiotic strains in two different types of biological fluids; simulated gastric juice and pancreatic secretion at 37 ° C after different contact times (5, 30 and 60 minutes). ^^ The results of survival in the presence of bile secretion were obtained by comparing the number of colonies growing in the specific medium "with" and "without" the addition of bile salts. | | | | | |

### Characterisation of the strain Lattobacillus paracasei (DSM 32258)

Strain: *Lattobacillus paracasei*
Internal identification number - ID: ID 1954
Probiotical commercial code: LMC-8
Deposit number in DSMZ international collection: DSM 32258

### Strain characterisation

### Origin

The strain *Lactobacillus paracasei* was isolated and analysed in the laboratories of Biolab Research srl, a subsidiary of the Mofin Alce Group, an affiliate of Probiotical S.p.A.

### Biochemical characterisation

### 1) Table 10 shows the sugar fermentation profile (API 50 CHL, bioMérieux)

**Table 10**

| **N.** | Sugars | Results | **N.** | Sugars | Results |
|---|---|---|---|---|---|
| **0** | Control | - | **25** | Esculin ferric citrate | + |
| **1** | Glycerol | - | **26** | Salicin | + |
| **2** | Erythritol | - | **27** | D-Cellobiose | + |
| **3** | D-Arabinose | - | **28** | D-Maltose | + |
| **4** | L-Arabinose | - | **29** | D-Lactose | - |
| **5** | D-Ribose | + | **30** | D-Melibiose | - |
| **6** | D-Xylose | - | **31** | D-Sucrose | + |
| **7** | L-Xylose | - | **32** | D-Trehalose | + |
| **8** | Adonitol | + | **33** | Inulin | + |
| **9** | Methyl-βD-Xylopyranoside | - | **34** | D-Melezitose | + |
| **10** | D-Galactose | + | **35** | D-Raffinose | - |
| **11** | D-Glucose | + | **36** | Starch | + |
| **12** | D-Fructose | + | **37** | Glycogen | - |
| **13** | D-Mannose | + | **38** | Xylitol | - |
| **14** | L-Sorbose | + | **39** | Gentiobiose | + |
| **15** | L-Rhamnose | - | **40** | D-Turanose | + |
| **16** | Dulcitol | - | **41** | D-Lyxose | + |
| **17** | Inositol | + | **42** | D-Tagatose | + |
| **18** | D-Mannitol | + | **43** | D-Fucose | - |
| **19** | D-Sorbitol | + | **44** | L-Fucose | - |
| **20** | Methyl-αD-Mannopyranoside | - | **45** | D-Arabitol | - |
| **21** | Methyl-αD-Glucopyranoside | + | **46** | L-Arabitol | - |
| **22** | N-Acetyl-Glucosamine | + | **47** | Potassium gluconate | + |
| **23** | Amygdalin | + | **48** | Potassium 2-Ketogluconate | - |
| **24** | Arbutin | + | **49** | Potassium 5-Ketogluconate | - |

### 2) Table 11 shows the enzymatic profile (API Zym, bioMérieux)

**Table 11**

| N. | Substrate | Enzymes analysed | Type of enzyme | Activity (nanomoles) |
|---|---|---|---|---|
| **1** | Negative control | - | - | - |
| **2** | 2-naphthyl-phosphate | Alkaline phosphatase | PHOSPHATASE | 10 |
| **3** | 2-naphthyl butyrate | Esterase (C 4) | LIPASE | - |
| **4** | 2-naphthyl caprylate | Esterase lipase (C 8) | | 5 |
| **5** | 2-naphthyl myristate | Lipase (C 14) | | - |
| **6** | L-leucyl-2-naphthylamide | Leucine arylamidase | PROTEASE | ≥40 |
| **7** | L-valyl-2-naphthylamide | Valine arylamidase | | ≥40 |
| **8** | L-cystyl-2-naphthylamide | Cystine arylamidase | | 20 |
| **9** | N-benzoyl-DL-arginine-2-naphthylamide | Trypsin | | - |
| **10** | N-glutaryl-phenylalanine-2-naphthylamide | α-chimotripsina | | 5 |
| **11** | 2-naphthyl-phosphate | Acid phosphatase | PHOSPHATASE | 30 |
| **12** | Naphthol-AS-BI-phosphate | Naphthol-AS-BI-phosphohydrolase | | 20 |
| **13** | 6-Br-2-naphthyl-αD-galactopyranoside | α-galactosidase | OXIDASE | - |
| **14** | 2-naphthyl-βD-galactopyranoside | β-galactosidase | | ≥40 |
| **15** | Naphthol-AS-BI-βD-glucoronide | β-glucuronidase | | - |
| **16** | 2-naphthyl-αD-glucopyranoside | α-glucosidase | | 30 |
| **17** | 6-Br-2-naphthyl-βD-glucopyranoside | β-glucosidase | | 20 |
| **18** | 1-naphthyl-N-acetyl-βD-glucosaminide | N-acetyl-β-glucosaminidase | | 5 |
| **19** | 6-Br-2-naphthyl-αD-mannopyranoside | α-mannosidase | | - |
| **20** | 2-naphthyl-αL-fucopyranoside | α-fucosidase | | 5 |

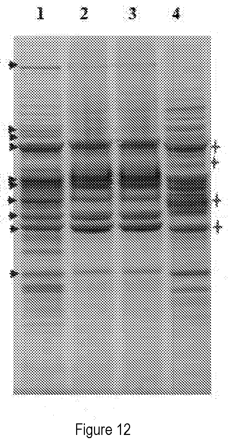

3) Figure 12 shows the protein profile (Electrophoresis on polyacrilamide gel), where:
1. Positive reference: *L.paracasei* LPC 01 ID 1119- CNCM I-1390
2. Strain: *L. paracasei* LMC-8 (ID 1954) - Master Cell Bank (origin)
3. Strain: *L. paracasei* LMC-8 (ID 1954) - Master Cell Bank (sub-culture 13#6)
4. Negative reference: *L. plantarum* LP01 ID 1171- LMG P-21021

Lines 2-3 show the protein profiles of the Master Cell Bank cultures of the strain; (see lines); in lines 1 and 2-3, the profile of the strain is characteristic of the species *Lactobacillus paracasei* (see arrows). As regards lines 4 and 2-3 the protein profile of the strain is different from the one obtained from a strain belonging to a different species (*Lactobacillus plantarum* LMG P-21021; see crosses).

### Molecular characterisation

### Species-specific classification:

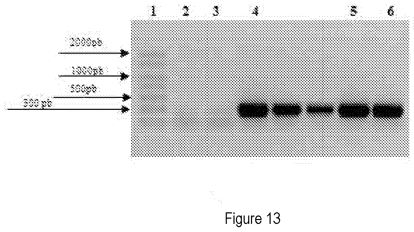

4) Figure 13 shows the Polymerase Chain Reaction (PCR) using the primers W2/Y2, where the reaction is positive for *Lactobacillus paracasei* and:
1. PCR Marker: Sigma 50-2000 bp
2. Blank: No DNA
3. Negative reference: *L. casei* DSM 20011
4. Positive reference: *L. paracasei* DSM 5622
5. Strain: *L. paracasei* LMC-8 ID 1954
6. Strain: *L. paracasei* LMC-8 ID 1954

### 5) 16S rDNA gene sequencing

| Length=1510 | | |
|---|---|---|
| Sequences producing significant alignments: | Score (Bits) | E Value |
| gb\|CP013921.1\| Lactobacillus paracasei strain KL1, complete g... | 2718 | 0.0 |
| gb\|KT159936.1\| Lactobacillus paracasei strain KF8 16S ribosom... | 2718 | 0.0 |
| gb\|KR816166.1\| Lactobacillus casei strain KF11 16S ribosomal ... | 2718 | 0.0 |
| gb\|KR816165.1\| Lactobacillus paracasei strain KF10 16S riboso... | 2718 | 0.0 |
| gb\|KR816160.1\| Lactobacillus paracasei strain KF1 16S ribosom... | 2718 | 0.0 |
| gb\|CP012148.1\| Lactobacillus paracasei strain L9, complete ge... | 2718 | 0.0 |
| gb\|CP012187.1\| Lactobacillus paracasei strain CAUH35, complet... | 2718 | 0.0 |
| gb\|CP001084.2\| Lactobacillus casei str. Zhang, complete genome | 2718 | 0.0 |
| enb\|HE983621.1\| Lactobacillus paracasei subsp. paracasei part... | 2718 | 0.0 |
| dbj\|LC096209.1\| Lactobacillus paracasei subsp. paracasei gene... | 2715 | 0.0 |

The results were obtained with the Blast program (http://blast.ncbi.nlm.nih.gov/Blast.cgi).
Score: Number used to evaluate the biological relevance of an identification. In sequence alignments, the score is a numerical value describing the overall quality of an alignment. Higher numbers correspond to greater similarity.
E-value (expected value): a value which, if correctly interpreted by a researcher, will indicate the likelihood of a score indicating a correlation between the two biological sequences. The lower the E-value, the more significant the score.

### Fingerprinting Profile

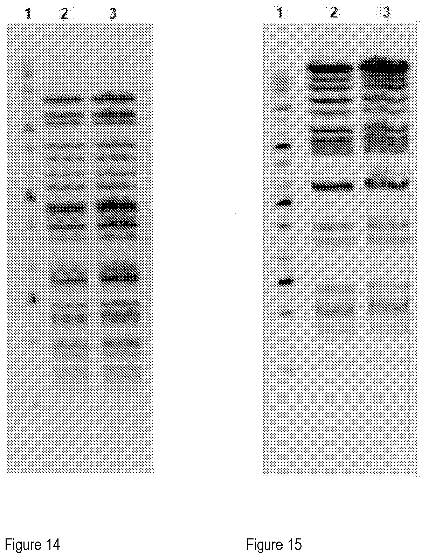

6) Figures 14 and 15 show the pulsed-field electrophoresis (PFGE) with the Notl enzyme(Figure 14) and Sfil enzyme (Figure 15), where:
1. Electrophoretic Marker: Sigma 50-1,000 kb
2. *L. paracasei* LMC-8 (ID 1954) - culture origin - Master Cell Bank
3. *L paracasei* LMC-8 (ID 1954) - 6th sub-culture - Master Cell Bank

### Biological characterisation

### 7) Table 12 shows the antibiotic resistance profile (E-test, ABBiodisk)

**Table 12**

| Antibiotic class | MIC* µg/ml | Bacterial strains | | | |
|---|---|---|---|---|---|
| | | *Lactobacillus paracasei* LMC-8 ID 1954 DSM 32258 | *Commercial Lactobacillus*^ | *Commercial Bifidobacterium* ^ | EFSA Limit 2012 |
| aminoglycosides | Gentamicin | 4 | 6 | 48 | 32 |
| | Streptomycin | 24 | 12 | 64 | 64 |
| | Kanamycin | 64 | 128 | n.d. | 64 |
| quinolones | Ciprofloxacin | 1.0 | 1 | 4 | - |
| glycopeptides | Vancomycin | >256 | >256 | 0.38 | n.r |
| lincosamides | Clindamycin | 0.094 | 0.50 | 0.047 | 1 |
| macrolides | Azithromycin | 0.75 | 0.38 | 0.75 | - |
| | Clarithromycin | 0.094 | 0.047 | 0.032 | - |
| | Erythromycin | 0.125 | 0.032 | 0.094 | 1 |
| oxazolidinone** | Linezolid | 1.5 | 2 | 0.38 | 4 |
| rifamicin | Rifampicin | 0.032 | 0.094 | 0.094 | - |
| strepogramin*** | Quinupristin/Dalfopristin | 0.25 | 1 | 0.25 | 4 |
| tetracyclines | Cloramphenicol | 2.0 | 3 | 0.75 | 4 |
| | Tetracycline | 0.38 | 0.50 | 8 | 4 |
| β-lactam | Amoxicillin | 0.50 | 0.50 | 0.064 | - |
| | Ampicillin | 0.38 | 0.50 | 0.016 | 4 |
| | Cefoxitin | >256 | >256 | 1 | - |
| | Cefuroxime | 1.0 | 1.5 | 0.25 | - |
| | Imipenem | 0.75 | 2 | 0.25 | - |

| | | | | | |
|---|---|---|---|---|---|
| ^ The commercial strains were used as a reference. The strains are not identified in this document for ethical reasons. The data are available on request * MIC (Minimum Inhibitory Concentration) assessment of the inhibition loop in agar with Etest strips (ABBiodisk). n.r. not required / n.d. not determined. ** included in EFSA 2005 *** included in EFSA 2008 | | | | | |

### 8) Table 13 shows the resistance to biological fluids (simulated gastric juice, simulated pancreatic juice and bile salts)

**Table 13**

| Strains | Biological fluids | *After different contact times (in minutes)*^ | | | *In the presence of bile in the medium^^* |
|---|---|---|---|---|---|
| | | 5' | 30' | 60' | |
| *Lattobacillus paracasei* LMC-8 ID 1954 DSM 32258 | Simulated gastric juice | 7 | 1 | 0 | |
| | Simulated pancreatic secretion | 21 | 21 | 15 | |
| | Bile salts | | | | 97 |
| Commercial Lactobacillus* | Simulated gastric juice | 90 | 30 | 19 | |
| | Simulated pancreatic secretion | 88 | 80 | 73 | |
| | Bile salts | | | | 55 |
| Commercial Bifidobacterium* | Simulated gastric juice | 90 | 65 | 25 | |
| | Simulated pancreatic secretion | 88 | 65 | 40 | |
| | Bile salts | | | | 4 |

| | | | | | |
|---|---|---|---|---|---|
| * The commercial strains were used as a reference. The strains are not identified in this document for ethical reasons. The data are available on request. ^ Table 13 shows the percentage of survival of the probiotic strains in two different types of biological fluids; simulated gastric juice and pancreatic secretion at 37 ° C after different contact times (5, 30 and 60 minutes). ^^ The results of survival in the presence of bile secretion were obtained by comparing the number of colonies growing in the specific medium "with" and "without" the addition of bile salts. | | | | | |

The present disclosure relates to at least one bacterial strain of human origin belonging to the genus *Lactobacillus* selected from the group comprising or, alternatively, consisting of:
1. *Lactobacillus plantarum* LMC1 (DSM 32252),
2. *Lactobacillus reuteri* LMC3 (DSM 32253),
3. *Lactobacillus paracasei* LMC4 (DSM 32254),
4. *Lactobacillus reuteri* LMC5 (DSM 32255),
5. *Lactobacillus rhamnosus* LMC6 (DSM 32256),
6. *Lactobacillus rhamnosus* LMC7 (DSM 32257),
7. *Lactobacillus paracasei* LMC8 (DSM 32258),
8. *Lactobacillus reuteri* LMC9 (DSM 32259),
9. *Lactobacillus rhamnosus* LMC10 (DSM 32260) and/or mixtures thereof (Group 1).

The present invention relates to at least one bacterial strain of human origin belonging to the genus *Lattobacillus* selected from the group comprising or, alternatively, consisting of:
1. *Lactobacillus plantarum* LMC1 (DSM 32252),
3. *Lactobacillus paracasei* LMC4 (DSM 32254),
5. *Lactobacillus rhamnosus* LMC6 (DSM 32256),
6. *Lactobacillus rhamnosus* LMC7 (DSM 32257),
7. *Lactobacillus paracasei* LMC8 (DSM 32258),
and/or mixtures thereof (Group 2).

Advantageously, the present invention relates to at least one bacterial strain of human origin belonging to the genus *Lattobacillus* selected from the group comprising or, alternatively, consisting of:
1. *Lactobacillus plantarum* LMC1 (DSM 32252),
3. *Lactobacillus paracasei* LMC4 (DSM 32254),
7. *Lactobacillus paracasei* LMC8 (DSM 32258),
and/or mixtures thereof (Group 3).

The bacterial strains of the present disclosure listed above were isolated from stool samples of healthy subjects, as described further below in the experimental section, and were all deposited with the DSMZ [*Deutsche Sammlung von Mikroorganismen und Zellkulturen GmbH,* Braunschweig, Germany) on 29.01.2016 and registered there under the respective accession numbers -DSM 32252-DSM 32260. Solely for the sake of convenience, the aforesaid strains may also be indicated in the description with their codes LMC1-3-4-5-6-7-8-9-10.

The present invention also relates to a pharmaceutical composition/formulation comprising a mixture which comprises or, alternatively, consists of at least one of the lactobacilli strains described above with their codes LMC1-3-4-5-6-7-8-9-10 and characterised, or else an appropriate combination thereof (for example, of two or more or all of them).

In one embodiment, the composition comprises a mixture which comprises or, alternatively, consists of at least one bacterial strain selected from among those of the group 2.

In another embodiment, the composition comprises a mixture which comprises or, alternatively, consists of at least one bacterial strain selected from among those of the group 3.

In said mixtures, the at least one bacterial strain or combination of lactobacilli strains of the invention is present in a total amount comprised from 1x10⁶ to 1x10¹² CFU/g of mixture; preferably, from 1x10⁷ to 1x10¹¹ CF/g of mixture; more preferably, from 1x10⁸ to 1x10¹⁰ CFU/g of mixture.

In said compositions, the at least one bacterial strain, or combination of the lactobacilli strains, of the invention, is present in a total amount comprised from 1x10⁶ to 1x10¹¹ CFU/g of composition; preferably, from 1x10⁷ to 1x10¹⁰ CF/g of composition; more preferably, from 1x10⁸ to 1x10⁹ CFU/g of composition.

Said compositions may also further comprise the necessary and/or appropriate amounts of co-formulants, excipients, carriers, surfactants, adjuvants, preservatives and colourants as desired.

Said substances are appropriately selected, in terms of quality and quantity, among those that are known and commonly used by the person skilled in the art of pharmaceutical formulations.

Said pharmaceutical composition may be supplied in the form that is best suited to the desired administration. This form can be any selected from among the pharmaceutical forms commonly known and prepared in the industry (for example, oral, topical, injectable). Solely by way of absolutely non-limiting example, with reference to a composition for oral administration, said composition can be in the form of a mouthwash, a tablet, a lozenge, a pastille, a pill, a capsule (with a soft or hard coating, controlled release), a powder or granules for sublingual administration or packaged in a sachet to be reconstituted, for example in water, prior to administration (according to need, said powder or granules can be incorporated in or coated by a suitable pharmaceutically acceptable polymer or mixture of polymers, able to impart particular properties to them, for example, gastro-resistance and/or a controlled/delayed release depending on the desired site of action in the body). Solely by way of absolutely non-limiting example, with reference to a composition for topical administration, said composition can be in the form of a gel, cream or ointment.

Said composition is prepared using well-known traditional technologies and production equipment (mixers, granulators, stirrers, packaging machines, and so forth) commonly used in the industry. The skilled person will have no difficulty, based solely on his knowledge, in identifying and selecting the one that is most suitable for the technical problem to be addressed in its preparation.

The previously described bacterial strains of the present invention (taken as such or in combination with one another), as well as the pharmaceutical formulations thereof, have shown to be effective, or at least very promising, agents capable of inhibiting and/or blocking and/or reducing the formation and/or growth of bacterial biofilms (in particular, those produced by pathogenic bacteria harmful to health), thereby enabling an optimal application of the necessary/desired antibacterial treatment (for example, antibiotics).

As is well known (19) and already mentioned earlier, bacterial biofilms play an often crucial role in human health, as they form a defensive barrier for the bacteria themselves against antibacterial therapies and other potential pathogens, as well as in infectious diseases where harmful bacteria invade normally sterile anatomical regions. As pointed out in the following experimental section, all the lactobacilli strains of the present invention were capable of inhibiting biofilm production by *S. aureus* (Table 1) to a significant degree, thus highlighting the crucial role of said lactobacilli against the pathogenic bacteria of clinical interest. In particular, in the example described, the bacterial strains LMC1-4-6-7-8 demonstrated the greatest inhibitory activity.

**Table 1**

| Supernatant | % of inhibition |
|---|---|
| LMC1 | 84 |
| LMC4 | 82 |
| LMC6 | 54 |
| LMC7 | 62 |
| LMC8 | 87 |

The bacterial strains of the present invention (Group 2 and/or Group 3) and the pharmaceutical compositions thereof have thus shown an excellent/good activity against bacterial biofilms, thanks to their ability to prevent and inhibit the formation and growth of the bacterial biofilms themselves. Therefore, the bacterial strains of the present invention and the pharmaceutical compositions thereof can be advantageously used in all health-related and non-health-related conditions in which there is an involvement of biofilms as the cause of greater microbial resistance to drugs, antibiotics, disinfectants and all other physical and chemical agents endowed with antimicrobial activity. In particular, the bacterial strains of the present invention (Group 2 and/or Group 3) and the pharmaceutical compositions thereof can be advantageously used, but not only:
- in the treatment of all superficial and deep infections in general, for example, but not limited to, surgical wounds and decubitus ulcers;
- in the treatment of all infections involving prostheses or the insertions of prostheses in bone tissues;
- in the treatment of infections from vascular and urinary catheters;
- in the treatment of infections from stents, cardiocirculatory devices, otologic, orthopaedic and dental prostheses, screws and nails;
- in the treatment of oral cavity infections (for example with an anti-biofilm and anti-plaque mouthwash), and infections of the oral and vaginal mucosa;
- in the treatment of the local infections (for example otitis, rhinosinusitis, pharyngitis, laryngitis and pneumonia) where a bacterial biofilm is involved as a persistent factor of infection;
- in laboratories to improve microbiological diagnosis when the bacteria are particularly adherent to tissues and prostheses, thus making the isolation and appropriate identification thereof difficult;
- in the field of healthcare in treatments for the removal of biofilms from surgical instruments and sanitary instruments in general (sanitisation);
- in the environmental, health and food sectors, in treatments for the removal and sanitisation of biofilms formed by *Legionella* or other harmful microorganisms, in water supply and sanitation systems and others;
- in the environmental and food sectors, in treatments for sanitising containers, vessels and containing tanks where a bacterial biofilm is present.

Therefore, the subject matter of the present invention further relates to the bacterial strains of the present invention and the pharmaceutical compositions thereof for use as a medication.

The subject matter of the present invention further relates to the bacterial strains of the present invention and the pharmaceutical compositions thereof for use as a medication in the treatment of health-related and non-health-related conditions in which there is an involvement of biofilms as the cause of greater microbial resistance to drugs, antibiotics, disinfectants and all other physical and chemical agents endowed with antimicrobial activity; in particular in the treatment of the health-related and non-health-related conditions described above by way of non-limiting example.

An experiment conducted with the lactobacilli of the present invention in order to verify their ability to inhibit biofilm formation by *Staphylococcus aureus* is described below solely by way of absolutely non-limiting example.

### MATERIALS AND METHODS

### Lactobacilli strains and Staphylococcus aureus

The nine lactobacilli strains of the disclosure (indicated in the present document as LMC1-3-4-5-6-7-8-9-10, as already mentioned earlier) used in this experiment were isolated in the Microbiology Laboratory of the IRCCS Istituto Ortopedico Galeazzi (Milan, Italy) from stool samples of healthy subjects, using a known methodology. The lactobacilli and strains of *S. aureus* were incubated overnight in a culture broth - Brain-Hearth infusion (BHI, bioMérieux, Marcy l'Etoile, France) - at 37°C under aerobiotic conditions.

### Evaluation of the anti-biofilm activity of the supernatants of the lactobacilli strains (Crystal Violet assay)

The anti-biofilm activity of the supernatants of the different lactobacilli strains was evaluated by quantifying the entity of biofilm produced by a methicillin-resistant strain of *S. aureus,* selected for its ability to produce biofilms, when incubated with the supernatants obtained from the tested lactobacilli strains. The amount of biofilm produced by *S. aureus* incubated in its supernatant was used as a negative control. Each strain of Lactobacillus was incubated in BHI for 24 hours, as previously described. Each culture medium was subsequently centrifuged at 6,000 rpm for 10 minutes in order to separate the bacterial cells from the supernatants. Then 20 µL of a 0.5 McFarland suspension of *S. aureus* were incubated in 180 µL of supernatant of the various lactobacilli in a new 96-well microplate. After 24 hours, the medium containing the non-adherent bacteria was removed and replaced with 180 µL of fresh supernatant. The plates were then incubated for 48 hours. Finally, the bacterial biofilm was evaluated by means of the spectrophotometric method described by Christensen et al. (18). The biofilm cultured in the 96-well plate was air dried and stained by immersion in a 5% Crystal Violet (CV) solution for 15 minutes and was subsequently dried again after numerous washes. The biomass of the biofilm was quantified by elution of the biofilm-CV bond with 3 ml of ethanol (96%) and subsequent measurement of the absorbance of 100 µL of the eluted solution at a wavelength of 595 nm by means of a microplate spectrophotometer (Multiskan TM FC; Thermo Scientific; Milan, Italy).

### Confocal Laser Scanning Microscope

A confocal laser scanning microscope was used to confirm the data obtained from the Crystal Violet analysis. The lactobacilli supernatants were used, whilst the biofilm produced by *S. aureus* in its own supernatant was used as a negative control. The microbial biofilm was incubated on MBEC Biofilm Inoculators (Innovotech Inc., Edmonton CA) plates according to the manufacturer's instructions. The wells were prepared with 20 µL of a 0.5 McFarland suspension of the supernatants of the different lactobacilli or the supernatant of the *S. aureus* strain (control). The supernatant was prepared as previously described. A FilmTracerTM LIVE/DEAD® Biofilm Viability Kit (Molecular Probes, Life Technologies Ltd., Paisley, UK) was used to reveal the biomass in the samples. All the samples were stained for 15 minutes in the dark at room temperature with an appropriate volume of a mixture of the two stains STYO9 and Propidium Iodide (PI) (3 µL of each element in 1 mL of saline solution). This allowed to distinguish the live bacteria from the dead ones. The stained biofilm was examined under the confocal microscope (Leica TCS SP5; Leica Microsystems CMS GmbH, Mannheim, Germany) using a 63X oil objective. A 488 nm laser was used to excite the SYTO9 stain, while the fluorescence emission was read between 500 nm and 540 nm. The PI stain was excited with a 561 nm laser, and the fluorescence emission thereof was read between 600 nm and 695 nm. A simultaneous acquisition of the two channels was carried out to minimise false colocalisation between the fluorescent spots due to movements of the bacteria. For each sample, 2 µm sequential optical sections were acquired and then put together in sequence on the z axis in order to obtain the complete thickness of the biofilm.

### RESULTS

### Evaluation of the anti-biofilm activity of the supernatant of the lactobacillus strains

A measurement was made of the quantity of biofilm produced by a methicillin-resistant strain of *S. aureus,* selected for its ability to produce biofilms (previously described), when incubated with the supernatant of the various lactobacillus strains. The obtained data (shown in the appended Table 1) showed the ability of the majority of the lactobacillus strains of the invention to significantly inhibit (> 50% of inhibition) the production of biofilm by *S. aureus.* In particular, when *S. aureus* was incubated with the supernatant, respectively, of LMC 1, 4,6,7 and 8, the quantity of biofilm produced proved to be greatly reduced. The strains LMC 1,4,6,7 and 8 showed to be the strains with the best activity of inhibiting the production of biofilm by *S. aureus.*

### Confocal Laser Scanning Microscope

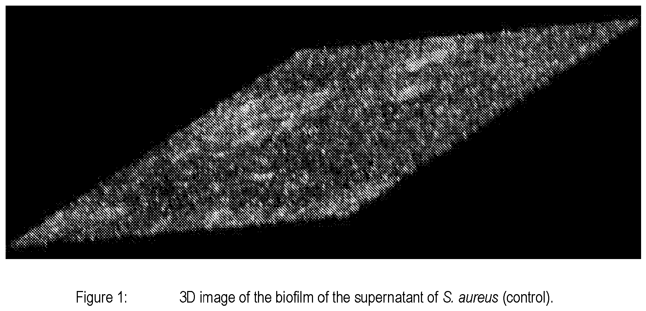

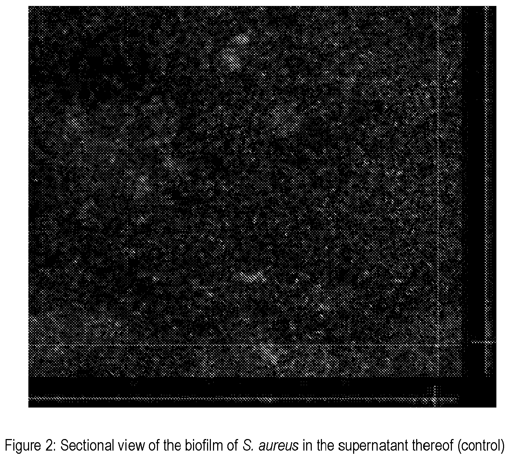

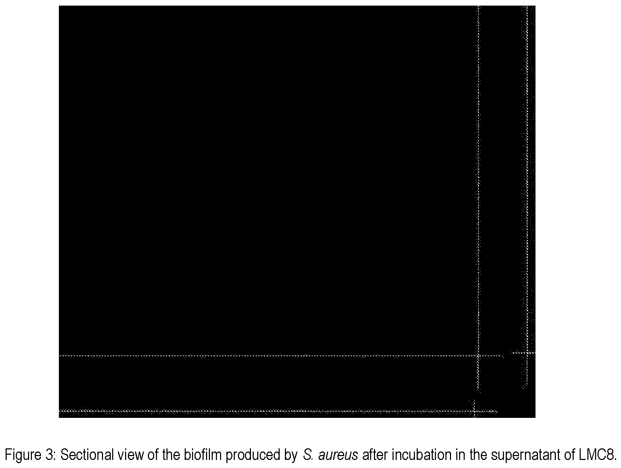

The strain LMC8, having the best anti-biofilm activity against the production of biofilm by *S. aureus* (see Table 1), was selected as the preferred example. Figure 1 represents a 3D reconstruction of the control sample, whereas Figure 2 represents a sectional view of said biofilm. Figure 1 and Figure 2 show the biofilm produced in the control sample (i.e. the biofilm of *S. aureus* produced in the supernatant of S. *aureus*). The biofilm produced by *S. aureus* is made up of live cells (green) and dead cells (red). Figure 3, on the other hand, represents the biofilm produced when *S. aureus* was incubated with the supernatant of LMC8. Comparing Figure 3 with Figures 1 and 2, it may be clearly observed that the production of biofilm by *S. aureus* was considerably reduced when the strain was incubated in the supernatant of LMC8. These data confirm the results previously obtained from the Crystal Violet assay. The same types of experiments, adopting substantially the same method and quantities similar to the ones previously described, were performed, *mutatis mutandis,* using the lactobacilli of the present disclosure, in particular the strain LMC8 and the strains LMC1, LMC4, LMC6, LMC7 and mixtures thereof, against biofilm formation by other bacterial strains harmful to health (for example *Candida, Escherichia coli, Klebsiella, Proteus mirabilis, Propionibacterium acnes*). The results obtained showed to be consistent with the ones described in the preceding experimental section for *S.aureus.*

### Industrial applicability

The lactobacilli of the present invention and the pharmaceutical compositions thereof have demonstrated to be excellent as agents endowed with anti-biofilm activity against bacterial biofilms, thanks to their ability to prevent and inhibit the formation and growth of the bacterial biofilms themselves. Therefore, the bacterial strains of the present invention and the pharmaceutical compositions thereof can be advantageously used in all health-related and non-health-related conditions in which there is an involvement of bacterial biofilms as the cause of greater microbial resistance to drugs, antibiotics, disinfectants and all other physical and chemical agents endowed with antimicrobial activity.

The bacterial strains of the present disclosure LMC-1 DSM 32252, LMC-3 DSM 32253, LMC-4 DSM 32254, LMC-5 DSM 32255, LMC-6 DSM 32256, LMC-7 DSM 32257, LMC-8 DSM 32258, LMC-9 DSM 32259, LMC-10 DSM 32260 were classified through phenotypic characterisation (API 50 CHL) and molecular identification with species-specific PCR.

### Phenotypic characterisation:

The API 50 CHL gallery (bioMèrieux code 50300) enables the study of carbohydrate metabolism in microorganisms.
It consists of 50 microtubes, the first of which, with no active ingredient, constitutes the negative control (blank). The subsequent microtubes each contain a well-defined amount of a dehydrated substrate belonging to the family of carbohydrates and derivatives (heterosides, polyols, uronic acids).
These substrates can be metabolised, which results in a change in colour: from purple/violet they turn to yellow, passing through various shades of green, due to a production of acid under anaerobiosis revealed by the pH indicator of the medium (Bromocresol Purple).

### Procedure:

The samples were centrifuged for 5' min at 10000 rpm in order to eliminate the culture medium and resuspended in 2ml of sterile distilled water. The inoculum was prepared in 5 ml of sterile distilled water with a quantity of sample equal to 2 on the McFarland scale (bioMèrieux). This suspension was inoculated in the ampoule of API 50 CHL Medium and immediately dispensed into the galleries. In order to assure the anaerobiosis of the sample, two drops of paraffin (bioMèrieux) were introduced for each dome and incubated in a temperature-controlled oven at 37°C.
A reading of the galleries was taken at different incubation times, at 24 and 48 hours.
A phenotypic classification of the various strains was obtained using APIWEB Plus software; the program computes a response, assigning a typicity index (T) and an identification percentage (%id), in addition to a comment on the quality of the analysis (Results in Table 15).
The use of the carbohydrate is manifested with a change in colour of the galleries: the reaction is positive if a bright green or yellow colour develops, and negative if dark green or purple develops. Checking the colour of the galleries is facilitated by a comparison with gallery n°. 0, which represents the blank.

### Molecular identification by species-specific PCR:

Within bacterial chromosome there are genes called 16s and 23s rRNA, which give rise to ribosome: this portion has variable regions (with the same sequence in bacteria of the same species).
By exploiting the specificity of the sequences belonging to the variable regions of rRNA, it is possible to design species-specific primers, which will lead to the amplification of the portion comprised between them.
During the thermal amplification cycle, the primers will be able to anneal only if the DNA template belongs to the species for which they were designed, yielding, as the amplification product, fragments whose dimensions will depend on the position of the oligonucleotides along the chromosomal DNA, and which will be longer the farther apart the primers are.

### Procedure:

The samples were processed according to MET_INT 049, current version.
The primer pairs used for classification are shown in Table 14:

**Table 14. List of primer pairs used for the PCR reaction**

| Bacterial species | Primer name | Nucleotide sequence | Ta °C | Product (pb) |
|---|---|---|---|---|
| *L. reuteri* | LFPR | CAG-ACT--AA-AGT-CTG-ACG-GT | 55 | 300 |
| | REU | AAC-ACT-CAA-GGA-TTG-TCT-GA | | |
| *L. paracasei sub.paracasei* | W2 | CACCGAGATTCAACATGG | 50 | 280 |
| | Y2 | CCCACTGCTGCCTCCCGTAGGAGT | | |
| *L plantarum* | P-REV | TCGGGATTACCAAACATCAC | 56 | 318 |
| | PLAN F | CCGTTTATGCGGAACACCTA | | |
| *L. rhamnosus* | RHA | GCG-ATG-CGA-ATT-TCT-ATT-ATT | 58 | 350(+160) |
| | PRI | CAG-ACT--AA-AGT-CTG-ACG-GT | | |

### Cell lysis

1 ml of the culture broth of each strain was centrifuged for 5' at 10000 rpm; the cells were subsequently resuspended in 1 ml of sterile water; 2µl of washed cells were added to 18 µl of Micro Lysis Buffer (Labogen) and the microtubes thus prepared were loaded into the thermocycler and subjected to a specific cell lysis cycle.

The lysed material was used as such for subsequent DNA amplification.
For each sample to be thermocycled, the PCR reaction was set up in sterile test tubes with the addition, in order, of 12.5µl of PCR Master Mix (Promega code M7502), sterile water q.s., primer 1 (100 µM - 0.3µl) and primer 2 (100 µM - 0.3µl) and 1µl of DNA, for a total volume of 25 µl.
For each PCR reaction, the following were introduced: a *positive control* consisting of DNA of the reference strain from an international collection and belonging to the same bacterial species as the sample undergoing analysis, a *negative control* consisting of DNA of the reference strain from an international collection but belonging to a bacterial species differing from the sample undergoing analysis and a blank consisting of the reaction buffer alone to check for any contamination.

Depending on the sample and the respective *annealing temperature* (Ta) specified in table 1, the samples were subjected to a thermal cycle for lactobacilli (MET-INT 049, current version).
The amplicates obtained were subjected to electrophoresis (30 min, 80V) in 1% agarose gel in 1X TAE buffer supplemented with ethidium bromide (5µl/10ml).
PCR 50-2000 bp Marker (SIGMA code P9577) was used to estimate the size of the amplified fragment.
At the end of the electrophoresis run, the agarose gel was viewed under ultraviolet light, using a transilluminator (Gel-Doc, BIO-RAD); results in Table 15.

### Results:

**Table 15. Results obtained from phenotypic and molecular characterisation.**

| Strain | API 50 CHL | Species-specific PCR |
|---|---|---|
| LMC-1 DSM 32252 | Excellent identification for *L.plantarum* %ID 99.6 T 0.96 | Positive result for *L.plantarum* |
| LMC-3 DSM 32253 | *Good identification for *L. fermentum* %ID 98.3 T 0.86 | Positive result for *L. reuteri* |
| LMC-4 DSM 32254 | Excellent identification for *L.paracasei ssp paracasei* %ID 99.3 T 0.68 | Positive result for *L.paracasei* |
| LMC-5 DSM 32255 | *Good identification for *L. fermentum* %ID 96.9 T 0.91 | Positive result for *L.reuteri* |
| LMC-6 DSM 32256 | Unidentified profile | Positive result for *L. rhamnosus* |
| LMC-7 DSM 32257 | Unidentified profile | Positive result for *L. rhamnosus* |
| LMC-8 DSM 32258 | Dubious profile for *L.paracasei ssp paracasei* %ID 99.7 T 0.56 | Positive result for *L. paracasei* |
| LMC-9 DSM 32259 | *Good identification for *L. fermentum* %ID 96.9 T 0.91 | Positive result for *L.reuteri* |
| LMC-10 DSM 32260 | Unacceptable profile | Positive result for *L. rhamnosus* |

| | | |
|---|---|---|
| * the species *L.reuteri* is not indicated in the APIWEB software, so the result obtained relates to the phylogenetically closest species; the result should in fact always be confirmed by species-specific PCR. | | |

### BIBLIOGRAPHY

1. Beikler T, Flemming T. Oral biofilm-associated diseases: trends and implications for quality of life, systemic health and expenditures. Periodontology 2000. 2011; 55:87-103.
2. Bjarnsholt T. The role of bacterial biofilms in chronic infections. APMIS Suppl. 2013;136:1-51.
3. Olson ME, Ceri H, Stremick, et al. Biofilm bacteria: formation and comparative susceptibility to antibiotics. Can J Vet Res. 2002;66:86-92.
4. Ceri H, Olson ME, Morck DW, et al. The MBEC assay system: Multiple equivalent biofilms for antibiotic and biocide susceptibility testing. Meth Enzymol. 2001;337:377-384.
5. Davies D. Understanding biofilm resistance to antibacterial agents. Nat Rev Drug Discov. 2003;2: 114-122.
6. Hung CS, Henderson JP. Emerging Concepts of Biofilms in Infectious Diseases. Mo Med. 2009; 106:292-296.
7. Li X, Kolltveit KM, Tronstad L, et al. Systemic diseases caused by oral infection. Clin Microbiol Rev. 2000; 13:547-558.
8. Bartzokas CA, Johnson R, Jane M, et al. Relation between mouth and haematogenous infection in total joint replacements. BMJ. 1994;309:506-508.
9. Mueller AA, Saldamli B, Stubringer S, et al. Oral bacterial cultures in nontraumatic brain abscesses: results of a first-line study. Oral Surg Oral Med Oral Pathol Oral Radiol Endod. 2009;107:469-476.
10. MylonasAl, Tzerbos FH, Mihalaki M, et al. Cerebral abscess of odontogenic origin. J Cranio-maxillofac Surg. 2007;35:63-67.
11. Parahitiyawa NB, Jin LJ, Leung WK, et al. Microbiology of odontogenic bacteremia: beyond endocarditis. Clin Microbiol Rev. 2009;22:46-64.
12. Ulivieri S, Olivieri G, Filosomi G. Brain abscess following dental procedures. Case report. Minerva Stomatol.. 2007;56:303-305.
13. Wagner KW, Schon R, Schumacher M, et al. Case report: brain and liver abscesses caused by oral infection with Streptococcus intermedius. Oral Surg Oral Med Oral Pathol Oral Radiol Endod. 2006; 102:e21-e23.
14. Chapple IL. The impact of oral disease upon systemic health-symposium overview. J Dent. 2009; 37:S568-S571.
15. Craig RG, Yip JK, So MK, et al. Relationship of destructive periodontal disease to the acute-phase response. J Periodontal. 2003;74:1007-1016.
16. Persson GR, Persson RE. Cardiovascular disease and periodontitis: an update on the associations and risk. J Clin Periodontol. 2008;35:362-379.
17. Jones SE, Versalovic J. Probiotic Lactobacillus reuteri biofilms produce antimicrobial and antiinflammatory factors. BMC Microbiol. 2009;9:35-43.
18. Christensen GD, Simpson WA, Younger JJ, et al. Adherence of coagulase-negative staphylococci to plastic tissue culture plates: a quantitative model for the adherence of staphylococci to medical devices. J Clin Microbiol. 1985;22:996-1006.
19. Galante J, Ho AC, Tingey S, Charalambous BM. Quorum Sensing and Biofilms in the Pathogen, Streptococcus Pneumoniae. Curr Pharm Des. 2014. [Epub ahead of print]
20. Biavati B., Scardovi V. and Moore W.E.C. Electrophoretic patterns of proteins in the genus Bifidobacterium and proposal of four new species. Int J Syst Bacteriol. 1982; 32: 358-373.
21. Burmeister M., Ulanovsky L. PULSE-FIELD GEL ELECTROPHORESIS: Protocol, Methods and Theories. Methods in Molecular Biology. 1992; vol 12.
22. Byun R., et al. Quantitative analysis of diverse Lactobacillus species present in advanced dental caries. J Clin Microbiol. 2004; 42(7):3128-3136.
23. Charteris W.P., et al. Development and application of an in vitro methodology to determine the transit tolerance of potentially probiotic Lactobacillus and Bifidobacterium species in the upper human gastrointestinal tract. J Appl Microbiol. 1998; 84(5):759-768.
24. Del Piano M., et al. In vitro sensitivity of probiotics to human pancreatic juice. J Clin Gastroenterol. 2008; 42(3): S170-173.
25. Desai A.R., et al. Discrimination of Dairy Industry Isolates of the Lactobacillus casei Group. J. Dairy Sci. 2006; 89:3345-3351.
26. Torriani S., et al. Differentiation of Lactobacillus plantarum, L. pentosus, and L. paraplantarum by recA Gene Sequence Analysis and Multiplex PCR Assay with recA Gene-Derived Primers. Appl Environ Microbiol. 2001; 67 (8): 3450-3454.
27. Tynkkynen S. et al. Comparison of Ribotyping, randomly amplified polymorphic DNA analysis and pulsed-field gel electrophoresis in typing of Lactobacillus rhamnosus and L. casei strains. Appl Environ Microb. 1999; 65 (9): 3908-3914.
28. Wall R., et al. Genomic diversity of cultivable Lactobacillus populations residing in the neonatal and adult gastrointestinal tract. FEMS Microbiol. Ecol. 2007; 59 (1) :127-137.
29. Walter J. et al. Detection and identification of gastrointestinal Lactobacillus Species by using denaturing gel electrophoresis and species-specific PCR primer. Appl Environ Microbiol. 2000; 66 (1): 297-303.
30. Aureli P, Fiore A, Scalfaro C, Franciosa G. Metodi microbiologici tradizionali e metodi molecolari per I'analisi degli integratori alimentari a base di o con probiotici per uso umano. Roma: Istituto Superiore di Sanità; 2008. (Rapporti ISTISAN 08/36)
31. Charteris W.P., Kelly P.M., Morelli L. Gradient diffusion antibiotic susceptibility testing of potentially probiotic lactobacilli. J Food Prot. 2001; 64(12): 2007-2014.
32. Danielsen M., Wind A. Susceptibility of Lactobacillus spp. to antimicrobial agents. Int J Food Microbiol. 2003; 82 (1): 1-11.
33. Egervärn M. et al. Antibiotic susceptibility profiles of Lactobacillus reuteri and Lactobacillus fermentum. J Food Prot. 2007; 70(2): 412-418.
34. Report from ISAPP Meeting on Antibiotic Resistance in Probiotic Bacteria. December 13, 2007 Paris.
35. The EFSA Journal. 2005; 223: 1-12. 2008; 732: 1-15. 2012; 10(6): 2740.

## Claims

1. A bacterial strain of human origin belonging to the genus Lactobacillus selected from the group consisting of:
*Lactobacillus plantarum,* deposit number DSM 32252 LMC1];
*Lactobacillus paracasei,* deposit number DSM 32254) [LMC4];
*Lactobacillus rhamnosus,* deposit number DSM 32256 [LMC6];
*Lactobacillus rhamnosus,* deposit number DSM 32257 [LMC7];
*Lactobacillus paracasei,* deposit number DSM 32258 [LMC8],
all deposited with the DSMZ [*Deutsche Sammlung von Mikroorganismen und Zellkulturen GmbH,* Braunschweig, Germany) on 29.01.2016.

2. The bacterial strain according to claim 1 for use as a medicament.

3. The bacterial strain according to claim 1 for use:
- in the treatment of superficial and deep infections, in the case of surgical wounds or decubitus ulcers;
- in the treatment of infections from vascular and urinary catheters;
- in the treatment of infections from stents, cardiocirculatory devices, prostheses, prosthetic insertions, otologic, orthopaedic and dental prostheses, screws and nails;
- in the treatment of oral cavity infections and infections of the oral and vaginal mucosa;
- in the treatment of local infections, otitis, rhinosinusitis, pharyngitis, laryngitis and pneumonia.

4. The bacterial strain for use according to claim 2, wherein the use is:
- in the treatment of superficial and deep infections, in the case of surgical wounds or decubitus ulcers;
- in the treatment of infections from vascular and urinary catheters;
- in the treatment of infections from stents, cardiocirculatory devices, prostheses, prosthetic insertions, otologic, orthopaedic and dental prostheses, screws and nails;
- in the treatment of oral cavity infections and infections of the oral and vaginal mucosa;
- in the treatment of local infections, otitis, rhinosinusitis, pharyngitis, laryngitis and pneumonia;

5. A composition comprising a mixture that comprises or, alternatively, consists of at least one bacterial strain selected from the group consisting of:
*Lactobacillus plantarum,* deposit number DSM 32252 [LMC1];
*Lactobacillus paracasei,* deposit number DSM 32254 [LMC4];
*Lactobacillus rhamnosus,* deposit number DSM 32256 [LMC6];
*Lactobacillus rhamnosus,* deposit number DSM 32257 [LMC7];
*Lactobacillus paracasei,* deposit number (DSM 32258 [LMC8],
all deposited with the DSMZ [*Deutsche Sammlung von Mikroorganismen und Zellkulturen GmbH,* Braunschweig, Germany) on 29.01.2016, and/or a combination thereof.

6. The composition according to claim 5, wherein said composition comprises a mixture that comprises or, alternatively, consists of at least one bacterial strain selected from the group consisting of:
*Lactobacillus plantarum* (DSM 32252) [LMC1];
*Lactobacillus paracasei* (DSM 32254) [LMC4];
*Lactobacillus paracasei* (DSM 32258) [LMC8], and/or a combination thereof.

7. The composition according to claims 5 or 6, wherein said composition is for use as a medicament.

8. The composition according to claims 5 or 6, for use:
- in the treatment of superficial and deep infections, in the case of surgical wounds or decubitus ulcers;
- in the treatment of infections from vascular and urinary catheters;
- in the treatment of infections from stents, cardiocirculatory devices, prostheses, prosthetic insertions, otologic, orthopaedic and dental prostheses, screws and nails;
- in the treatment of oral cavity infections and infections of the oral and vaginal mucosa;
- in the treatment of local infections, otitis, rhinosinusitis, pharyngitis, laryngitis and pneumonia.

9. The composition for use according to claim 7, wherein the use is:
- in the treatment of superficial and deep infections, in the case of surgical wounds or decubitus ulcers;
- in the treatment of infections from vascular and urinary catheters;
- in the treatment of infections from stents, cardiocirculatory devices, prostheses, prosthetic insertions, otologic, orthopaedic and dental prostheses, screws and nails;
- in the treatment of oral cavity infections and infections of the oral and vaginal mucosa;
- in the treatment of local infections, otitis, rhinosinusitis, pharyngitis, laryngitis and pneumonia.

10. Use of the composition according to claims 5-6:
- in the *in-vitro* treatment of bacterial biofilms particularly adherent to tissues and prostheses in laboratories, so as to improve the possibility of microbiological diagnosis of the same;
- in the *in-vitro* treatments for biofilm removal from surgical instruments and sanitary instruments in general (sanitisation) in the field of healthcare;
- in the *in-vitro* treatments for the removal and sanitisation of biofilms formed by Legionella or other harmful microorganisms in the environmental and food sectors, in water supply and sanitation systems;
- in treatments for cleaning containers, vessels and containing tanks where a bacterial biofilm is present, in the environmental and food sectors;
- in the *in-vitro* treatment of a bacterial biofilm that has formed due to a greater microbial resistance to drugs, antibiotics, disinfectants and physical and chemical agents endowed with antimicrobial activity.

11. The composition according to claims 5 or 6, the composition for use according to claims 7, 8 or 9, the use according to claim 10, wherein said at least one bacterial strain contained in said mixture is present in a total amount comprised from 1x10⁶ to 1x10¹² CFU/g of mixture; preferably, from 1x10⁷ to 1x10¹¹ CF/g of mixture; more preferably, from 1x10⁸ to 1x10¹⁰ CFU/g of mixture.

12. The composition according to claim 5 or 6, the composition for use according to claim 7, 8 or 9, the use according to claim 10, wherein said at least one bacterial strain contained in said composition is present in a total amount comprised from 1x10⁶ to 1x10¹¹ CFU/g of composition; preferably, from 1x10⁷ to 1x10¹⁰ CF/g of composition; more preferably, from 1x10⁸ to 1x10⁹ CFU/g of composition.

13. The composition according to claim 5, 6, 11, or 12, the composition for use according to claim 7, 8 or 9, the use according to claim 10, wherein said composition is active against the formation of biofilms by harmful bacterial strains such as, preferably, ones selected from among *Candida, Escherichia coli, Klebsiella, Proteus mirabilis* and *Propionibacterium acnes, S.aureus.*

## Patentansprüche

1. Bakterienstamm menschlichen Ursprungs, gehörend zur Gattung Lactobacillus, ausgewählt aus der Gruppe, bestehend aus:
*Lactobacillus plantarum,* Hinterlegungsnummer DSM 32252 [LMC1] ;
*Lactobacillus paracasei,* Hinterlegungsnummer DSM 32254 [LMC4] ;
*Lactobacillus rhamnosus,* Hinterlegungsnummer DSM 32256 [LMC6] ;
*Lactobacillus rhamnosus,* Hinterlegungsnummer DSM 32257 [LMC7] ;
*Lactobacillus paracasei,* Hinterlegungsnummer DSM 32258 [LMC8],
alle am 29.01.2016 bei der DSMZ (Deutschen Sammlung von Mikroorganismen und Zellkulturen GmbH, Braunschweig, Deutschland) hinterlegt.

2. Bakterienstamm gemäß Anspruch 1 zur Verwendung als Arzneimittel.

3. Bakterienstamm gemäß Anspruch 1 zur Verwendung:
- bei der Behandlung von oberflächlichen und tiefen Infektionen, im Fall von Operationswundern oder Dekubitalgeschwüren;
- bei der Behandlung von Infektionen durch Gefäß- und Harnkatheter;
- bei der Behandlung von Infektionen durch Stents, Herz-Kreislauf-Geräte, Prothesen, Protheseneinlagen, otologische, orthopädische und zahnmedizinische Prothesen, Schrauben und Nägel;
- bei der Behandlung von Infektionen des Mundraums und Infektionen der Mund- und Vaginalschleimhaut;
- bei der Behandlung von lokalen Infektionen, Otitis, Rhinosinusitis, Pharyngitis, Laryngitis und Lungenentzündung.

4. Bakterienstamm zur Verwendung gemäß Anspruch 2, worin die Verwendung
- bei der Behandlung von oberflächlichen und tiefen Infektionen, im Fall von Operationswundern oder Dekubitalgeschwüren;
- bei der Behandlung von Infektionen durch Gefäß- und Harnkatheter;
- bei der Behandlung von Infektionen durch Stents, Herz-Kreislauf-Geräte, Prothesen, Protheseneinlagen, otologische, orthopädische und zahnmedizinische Prothesen, Schrauben und Nägel;
- bei der Behandlung von Infektionen des Mundraums und Infektionen der Mund- und Vaginalschleimhaut;
- bei der Behandlung von lokalen Infektionen, Otitis, Rhinosinusitis, Pharyngitis, Laryngitis und Lungenentzündung, ist.

5. Zusammensetzung, umfassend ein Gemisch, das umfasst oder alternativ besteht aus mindestens einem Bakterienstamm, ausgewählt aus der Gruppe, bestehend aus:
*Lactobacillus plantarum,* Hinterlegungsnummer DSM 32252 [LMC1] ;
*Lactobacillus paracasei,* Hinterlegungsnummer DSM 32254 [LMC4] ;
*Lactobacillus rhamnosus,* Hinterlegungsnummer DSM 32256 [LMC6] ;
*Lactobacillus rhamnosus,* Hinterlegungsnummer DSM 32257 [LMC7] ;
*Lactobacillus paracasei,* Hinterlegungsnummer DSM 32258 [LMC8],
alle am 29.01.2016 bei der DSMZ (Deutschen Sammlung von Mikroorganismen und Zellkulturen GmbH, Braunschweig,
Deutschland) hinterlegt, und/oder eine Kombination davon.

6. Zusammensetzung gemäß Anspruch 5, worin die Zusammensetzung ein Gemisch umfasst, das umfasst oder alternativ besteht aus mindestens einem Bakterienstamm, ausgewählt aus der Gruppe, bestehend aus:
*Lactobacillus plantarum* (DSM 32252) [LMC1];
*Lactobacillus paracasei* (DSM 32254) [LMC4];
*Lactobacillus paracasei* (DSM 32258) [LMC8], und/oder eine Kombination davon

7. Zusammensetzung gemäß Anspruch 5 oder 6, worin die Zusammensetzung zur Verwendung als Arzneimittel bestimmt ist.

8. Zusammensetzung gemäß Anspruch 5 oder 6, zur Verwendung:
- bei der Behandlung von oberflächlichen und tiefen Infektionen, im Fall von Operationswundern oder Dekubitalgeschwüren;
- bei der Behandlung von Infektionen durch Gefäß- und Harnkatheter;
- bei der Behandlung von Infektionen durch Stents, Herz-Kreislauf-Geräte, Prothesen, Protheseneinlagen, otologische, orthopädische und zahnmedizinische Prothesen, Schrauben und Nägel;
- bei der Behandlung von Infektionen des Mundraums und Infektionen der Mund- und Vaginalschleimhaut;
- bei der Behandlung von lokalen Infektionen, Otitis, Rhinosinusitis, Pharyngitis, Laryngitis und Lungenentzündung.

9. Zusammensetzung zur Verwendung gemäß Anspruch 7, worin die Verwendung
- bei der Behandlung von oberflächlichen und tiefen Infektionen, im Fall von Operationswundern oder Dekubitalgeschwüren;
- bei der Behandlung von Infektionen durch Gefäß- und Harnkatheter;
- bei der Behandlung von Infektionen durch Stents, Herz-Kreislauf-Geräte, Prothesen, Protheseneinlagen, otologische, orthopädische und zahnmedizinische Prothesen, Schrauben und Nägel;
- bei der Behandlung von Infektionen des Mundraums und Infektionen der Mund- und Vaginalschleimhaut;
- bei der Behandlung von lokalen Infektionen, Otitis, Rhinosinusitis, Pharyngitis, Laryngitis und Lungenentzündung, ist.

10. Verwendung der Zusammensetzung gemäß den Ansprüchen 5-6:
- bei der *in-vitro*-Behandlung von bakteriellen Biofilmen, die insbesondere an Geweben oder Prothesen in Laboren haften, um die Möglichkeit der mikrobiologischen Diagnose derselben zu verbessern;
- bei den *in-vitro*-Behandlungen zur Biofilmentfernung von chirurgischen Instrumenten und Sanitärinstrumenten im Allgemeinen (Sanitisierung) im Bereich des Gesundheitswesens;
- bei den *in-vitro*-Behandlungen zur Entfernung und Sanitisierung von Biofilmen, die durch Legionellen oder andere schädliche Mikroorganismen im Umwelt- und Lebensmittelbereich, in Wasserversorgungs- und Abwassersystemen gebildet werden;
- bei den Behandlungen zur Reinigung von Behältern, Gefäßen und enthaltenden Tanks, in denen ein bakterieller Biofilm vorhanden ist, im Umwelt- und Lebensmittelbereich;
- bei der *in-vitro*-Behandlung eines bakteriellen Biofilms, der sich aufgrund einer größeren mikrobiellen Resistenz gegen Arzneimittel, Antibiotika, Desinfektionsmittel und physikalische und chemische Mittel mit antimikrobieller Wirkung gebildet hat.

11. Zusammensetzung gemäß Anspruch 5 oder 6, Zusammensetzung zur Verwendung gemäß Anspruch 7, 8 oder 9, Verwendung gemäß Anspruch 10, worin der mindestens eine im Gemisch enthaltene Bakterienstamm in einer Gesamtmenge von 1x10⁶ bis 1x10¹² CFU/g des Gemischs; bevorzugt von 1x10⁷ bis 1x10¹¹ CFU/g des Gemischs; stärker bevorzugt von 1x10⁸ bis 1x10¹⁰ CFU/g des Gemischs, vorhanden ist.

12. Zusammensetzung gemäß Anspruch 5 oder 6, Zusammensetzung zur Verwendung gemäß Anspruch 7, 8 oder 9, Verwendung gemäß Anspruch 10, worin der mindestens eine im Gemisch enthaltene Bakterienstamm in einer Gesamtmenge von 1x10⁶ bis 1x10¹¹ CFU/g des Gemischs; bevorzugt von 1x10⁷ bis 1x10¹⁰ CFU/g des Gemischs; stärker bevorzugt von 1x10⁸ bis 1x10⁹ CFU/g des Gemischs, vorhanden ist.

13. Zusammensetzung gemäß Anspruch 5, 6, 11 oder 12 Zusammensetzung zur Verwendung gemäß Anspruch 7, 8 oder 9, Verwendung gemäß Anspruch 10, worin die Zusammensetzung gegen die Bildung von Biofilmen durch schädliche Bakterienstämme, wie bevorzugt solche, ausgewählt aus *Candida, Escherichia coli, Klebsiella, Proteus mirabilis* und *Propionibacterium acnes, S. aureus,* wirksam ist.

## Revendications

1. Souche bactérienne d'origine humaine appartenant au genre Lactobacillus, choisie dans le groupe constitué par :
*Lactobacillus plantarum,* numéro de dépôt DSM 32252 [LMC1] ;
*Lactobacillus paracasei,* numéro de dépôt DSM 32254 [LMC4] ;
*Lactobacillus rhamnosus,* numéro de dépôt DSM 32256 [LMC6] ;
*Lactobacillus rhamnosus,* numéro de dépôt DSM 32257 [LMC7] ;
*Lactobacillus paracasei,* numéro de dépôt DSM 32258 [LMC8],
toutes déposées au DMSZ [*Deutsche Sammlung von Mikroorganismen und Zellkulturen GmbH,* Braunschweig, Allemagne) le 29 janvier 2016.

2. Souche bactérienne selon la revendication 1, pour une utilisation en tant que médicament.

3. Souche bactérienne selon la revendication 1, pour une utilisation :
- dans le traitement d'infections superficielles et profondes, dans le cas de plaies chirurgicales ou d'ulcères de décubitus ;
- dans le traitement d'infections dues à des cathéters vasculaires et urinaires ;
- dans le traitement d'infections dues à des endoprothèses vasculaires, des dispositifs cardio-circulatoires, des prothèses, des insertions prosthétiques, des prothèses otologiques, orthopédiques et dentaires, des vis et des clous ;
- dans le traitement d'infections de la cavité orale et d'infections de la muqueuse orale et vaginale ;
- dans le traitement d'infections locales, d'une otite, d'une rhinosinusite, d'une pharyngite, d'une laryngite et d'une pneumonie.

4. Souche bactérienne pour une utilisation selon la revendication 2, dans laquelle l'utilisation est :
- dans le traitement d'infections superficielles et profondes, dans le cas de plaies chirurgicales ou d'ulcères de décubitus ;
- dans le traitement d'infections dues à des cathéters vasculaires et urinaires ;
- dans le traitement d'infections dues à des endoprothèses vasculaires, des dispositifs cardio-circulatoires, des prothèses, des insertions prosthétiques, des prothèses otologiques, orthopédiques et dentaires, des vis et des clous ;
- dans le traitement d'infections de la cavité orale et d'infections de la muqueuse orale et vaginale ;
- dans le traitement d'infections locales, d'une otite, d'une rhinosinusite, d'une pharyngite, d'une laryngite et d'une pneumonie.

5. Composition comprenant un mélange qui comprend ou, en variante, consiste en, au moins une souche bactérienne choisie dans le groupe constitué par :
*Lactobacillus plantarum,* numéro de dépôt DSM 32252 [LMC1] ;
*Lactobacillus paracasei,* numéro de dépôt DSM 32254 [LMC4] ;
*Lactobacillus rhamnosus,* numéro de dépôt DSM 32256 [LMC6] ;
*Lactobacillus rhamnosus,* numéro de dépôt DSM 32257 [LMC7] ;
*Lactobacillus paracasei,* numéro de dépôt DSM 32258 [LMC8],
toutes déposées au DMSZ [*Deutsche Sammlung von Mikroorganismen und Zellkulturen GmbH,* Braunschweig, Allemagne) le 29 janvier 2016 et/ou une de leurs combinaisons.

6. Composition selon la revendication 5, laquelle composition comprenant un mélange qui comprend ou, en variante, consiste en, au moins une souche bactérienne choisie dans le groupe constitué par :
*Lactobacillus plantarum,* numéro de dépôt DSM 32252 [LMC1] ;
*Lactobacillus paracasei,* numéro de dépôt DSM 32254 [LMC4] ;
*Lactobacillus paracasei,* numéro de dépôt DSM 32258 [LMC8],
et/ou une de leurs combinaisons.

7. Composition selon la revendication 5 ou 6, laquelle composition est destinée à être utilisée en tant que médicament.

8. Composition selon la revendication 5 ou 6, pour une utilisation :
- dans le traitement d'infections superficielles et profondes, dans le cas de plaies chirurgicales ou d'ulcères de décubitus ;
- dans le traitement d'infections dues à des cathéters vasculaires et urinaires ;
- dans le traitement d'infections dues à des endoprothèses vasculaires, des dispositifs cardio-circulatoires, des prothèses, des insertions prosthétiques, des prothèses otologiques, orthopédiques et dentaires, des vis et des clous ;
- dans le traitement d'infections de la cavité orale et d'infections de la muqueuse orale et vaginale ;
- dans le traitement d'infections locales, d'une otite, d'une rhinosinusite, d'une pharyngite, d'une laryngite et d'une pneumonie.

9. Composition pour une utilisation selon la revendication 7, dans laquelle l'utilisation est :
- dans le traitement d'infections superficielles et profondes, dans le cas de plaies chirurgicales ou d'ulcères de décubitus ;
- dans le traitement d'infections dues à des cathéters vasculaires et urinaires ;
- dans le traitement d'infections dues à des endoprothèses vasculaires, des dispositifs cardio-circulatoires, des prothèses, des insertions prosthétiques, des prothèses otologiques, orthopédiques et dentaires, des vis et des clous ;
- dans le traitement d'infections de la cavité orale et d'infections de la muqueuse orale et vaginale ;
- dans le traitement d'infections locales, d'une otite, d'une rhinosinusite, d'une pharyngite, d'une laryngite et d'une pneumonie.

10. Utilisation de la composition selon les revendications 5 et 6 :
- dans le traitement in vitro de biofilms bactériens particulièrement adhérents à des tissus et prothèses en laboratoire, de manière à améliorer la possibilité d'un diagnostic microbiologique de ceux-ci ;
- dans les traitements in vitro pour l'élimination de biofilms sur des instruments chirurgicaux et des instruments sanitaires en général (désinfection) dans le domaine de la santé ;
- dans les traitements in vitro pour l'élimination et la désinfection de biofilms formés par Legionella ou d'autres microorganismes dangereux dans les secteurs environnemental et alimentaire, dans les systèmes d'approvisionnement en eau et d'assainissement ;
- dans les traitements pour nettoyer des récipients, cuves et réservoirs lorsqu'un biofilm bactérien est présent, dans les secteurs environnemental et alimentaire ;
- dans le traitement in vitro d'un biofilm bactérien qui s'est formé à cause d'une plus forte résistance microbienne aux médicaments, antibiotiques, désinfectants et agents physiques et chimiques dotés d'une activité antimicrobienne.

11. Composition selon la revendication 5 ou 6, composition pour une utilisation selon la revendication 7, 8 ou 9, utilisation selon la revendication 10, dans laquelle ladite au moins une souche bactérienne contenue dans ledit mélange est présente en une quantité totale de 1 x 10⁶ à 1 x 10¹² CFU/g de mélange ; de préférence de 1 x 10⁷ à 1 x 10¹¹ CFU/g de mélange ; mieux encore de 1 x 10⁸ à 1 x 10¹⁰ CFU/g de mélange.

12. Composition selon la revendication 5 ou 6, composition pour une utilisation selon la revendication 7, 8 ou 9, utilisation selon la revendication 10, dans laquelle ladite au moins une souche bactérienne contenue dans ladite composition est présente en une quantité totale de 1 x 10⁶ à 1 x 10¹¹ CFU/g de composition ; de préférence de 1 x 10⁷ à 1 x 10¹⁰ CFU/g de composition ; mieux encore de 1 x 10⁸ à 1 x 10⁹ CFU/g de composition.

13. Composition selon la revendication 5, 6, 11 ou 12, composition pour une utilisation selon la revendication 7, 8 ou 9, utilisation selon la revendication 10, dans laquelle ladite composition est active contre la formation de biofilms par des souches bactériennes dangereuses telles que, de préférence, celles choisies parmi *Candida, Escherichia coli, Klebsiella, Proteus mirabilis* et *Propionibacterium acnes, S. aureus.*
